(19) Europäisches Patentamt
European Patent Office
Office européen des brevets

(11) EP 1 272 527 B2

(12) # NOUVEAU FASCICULE DE BREVET EUROPEEN
Après la procédure d'opposition

(45) Date de publication et mention de la décision concernant l'opposition:
**29.08.2018 Bulletin 2018/35**

(45) Mention de la délivrance du brevet:
**31.12.2008 Bulletin 2009/01**

(21) Numéro de dépôt: **01927974.4**

(22) Date de dépôt: **12.04.2001**

(51) Int Cl.:
***C07K 16/34*** (2006.01) ***C12N 15/13*** (2006.01)
***C12N 5/20*** (2006.01) ***A61K 39/395*** (2006.01)
***G01N 33/577*** (2006.01) ***A61P 37/02*** (2006.01)
***A61P 35/00*** (2006.01) ***A61P 31/00*** (2006.01)

(86) Numéro de dépôt international:
**PCT/FR2001/001127**

(87) Numéro de publication internationale:
**WO 2001/077181 (18.10.2001 Gazette 2001/42)**

(54) **PREPARATION D'ANTICORPS AYANT UNE ADCC VIA LE CD16, NOTAMMENT DES ANTICORPS MONOCLONAUX ANTI RHESUS D**

VERFAHREN VON ANTIKÖRPER MIT ADCC VIA CD16, Z.B. MONOKLONALE ANTIKÖRPER GEGEN RHESUS D

PREPARATION OF ANTIBODIES HAVING ADCC VIA CD16, SUCH AS ANTI-RHESUS D MONOCLONAL ANTIBODIES

(84) Etats contractants désignés:
**AT BE CH CY DE DK ES FI FR GB GR IE IT LI LU MC NL PT SE TR**

(30) Priorité: **12.04.2000 FR 0004685**

(43) Date de publication de la demande:
**08.01.2003 Bulletin 2003/02**

(60) Demande divisionnaire:
**04028814.4 / 1 518 864**
**10180551.3 / 2 341 078**
**15173391.2 / 2 947 099**

(73) Titulaire: **Laboratoire Français du Fractionnement et des Biotechnologies**
**91940 Les Ulis (FR)**

(72) Inventeurs:
- **BELIARD, Roland**
  **F-59211 Santes (FR)**
- **BOUREL, Dominique**
  **F-59110 La Madeleine (FR)**
- **GLACET, Arnaud**
  **F-59147 Gondecourt (FR)**
- **DE ROMEUF, Christophe**
  **59000 Lille (FR)**
- **BIHOREAU, Nicolas**
  **91400 Orsay (FR)**
- **NONY, Emmanuel**
  **92160 ANTONY (FR)**

(74) Mandataire: **Regimbeau**
**20, rue de Chazelles**
**75847 Paris Cedex 17 (FR)**

(56) Documents cités:
**EP-A1- 1 176 195 WO-A1-00/61739**
**FR-A- 2 653 561 US-A- 5 851 524**

- **KLEIN PHILIPPE ET AL: "Human recombinant anti-Rh(D) monoclonal antibodies: Improvement of biological properties by in vitro class-switch." HUMAN ANTIBODIES, vol. 8, no. 1, 1997, pages 17-25, XP001015726 ISSN: 1093-2607**
- **HADLEY, A. G. ET AL: "The functional activity of Fc.gamma.RII and Fc.gamma.RIII on subsets of human lymphocytes" IMMUNOLOGY (1992), 76(3), 446-51 , XP001015733**
- **BREDIUS R G M ET AL: "Role of neutrophil Fc-gamma-RIIa (CD32) and Fc-gamma-RIIIb (CD16) polymorphic forms in phagocytosis of human IgG1- and IgG3-opsonized bacteria and erythrocytes." IMMUNOLOGY, vol. 83, no. 4, 1994, pages 624-630, XP000971450 ISSN: 0019-2805**
- **PATERSON T ET AL: "Variation in IgG1 heavy chain allotype does not contribute to differences in biological activity of two human anti-Rhesus (D) monoclonal antibodies" IMMUNOTECHNOLOGY,NL,ELSEVIER SCIENCE PUBLISHERS BV, vol. 4, no. 1, 1 juin 1998 (1998-06-01), pages 37-47, XP004127385 ISSN: 1380-2933**

EP 1 272 527 B2

- UGEN K E ET AL: "New recombinant multimer of two fusion proteins, each including part of C4 binding protein;recombinant complement-C4 binding protein and CD4, CD8, CD16, CD35, Rhesus-D, antigen, enzyme or single chain antibody fusion protein multimer for use as a recombinant vaccine or diagnostic" VACCINE,GB,BUTTERWORTH SCIENTIFIC. GUILDFORD, vol. 15, no. 8, 1 juin 1997 (1997-06-01), page 935 XP004075692 ISSN: 0264-410X
- BIHOREAU N ET AL: "Combination of capillary electrophoresis and matrix-assisted laser desorption ionization mass spectrometry for glycosylation analysis of a human monoclonal anti-Rhesus(D) antibody" JOURNAL OF CHROMATOGRAPHY B: BIOMEDICAL SCIENCES & APPLICATIONS,NL,ELSEVIER SCIENCE PUBLISHERS, vol. 697, no. 1-2, 12 septembre 1997 (1997-09-12), pages 123-133, XP004090752 ISSN: 0378-4347
- DE ROMEUF C. ET AL: 'Expression of human anti-Rh (D) monoclonal antibodies into different cell lines: influence on their functional properties' ANTIBODIES : PRODUCTION AND PURIFICATION vol. 1, 2004,
- SHINKAWA T. ET AL: 'The absence of fucose but not the presence of galactose or bisecting N-acetylglucosamine of human IgG1 complex-type oligosaccharides shows the critical role of enhancing antibody-dependent cellular cytotoxicity' THE JOURNAL OF BIOLOGICAL CHEMISTRY vol. 278, no. 5, 31 Janvier 2003, pages 3466 - 3473
- LIFELY R. ET AL: 'Glycosylation and biological activity of CAMPATH-1H expressed in different cell lines and grown under different culture conditions' GLYCOBIOLOGY vol. 5, no. 8, 1995, pages 813 - 822
- WRIGHT A. ET AL: 'Effect of glycosylation on antibody function: implications for genetic engineering' TIBTECH vol. 15, Janvier 1997, pages 26 - 32
- JEFFERIS R. ET AL: 'IgG-Fc-mediated effector functions: molecular definition of interaction sites for effector ligands and the role of glycosylation' IMMUNOLOGICAL REVIEWS vol. 163, 1998, pages 59 - 76
- CANT D. ET AL: 'Glycosylation and functional activity of anti-D secreted by two human lymphoblastoid cell lines' CYTOTECHNOLOGY vol. 15, 1994, pages 223 - 228
- UMANA P. ET AL: 'Engineered glycoforms of an antineuroblastoma IgG1 with optimized antibody-dependent cellular cytotoxic activity' NATURE BIOTECHNOLOGY vol. 17, Février 1999, pages 176 - 180

## Description

La présente invention concerne un procédé d'obtention et de sélection d'anticorps monoclonaux par un test de type ADCC, lesdits anticorps pouvant activer les récepteurs Fcγ de type III. Des anticorps monoclonaux présentant une structure glycannique particulière, les cellules produisant lesdits anticorps, les méthodes de préparation des cellules productrices, ainsi que les compositions pharmaceutiques ou les tests diagnostiques comprenant lesdits anticorps sont décrits ci-après. Les anticorps anti-D peuvent être utilisés pour la prévention de l'isoimmunisation Rhésus d'individus Rh négatifs, notamment de la maladie hémolytique du nouveau-né (MHNN) ou dans des applications comme le Purpura Thrombocytopénique Idiopathique (PTI).

L'immunothérapie passive à l'aide d'anticorps polyclonaux est réalisée depuis les années 1970. Cependant, la fabrication des immunoglobulines polyclonales pose plusieurs problèmes :

> L'immunisation de sujets volontaires a été interrompue en France en 1997, en raison des problèmes éthiques que présentent de tels actes. En France, comme en Europe, le nombre de donneurs immunisés est trop faible pour assurer un approvisionnement suffisant en certains anticorps si bien qu'il s'avère nécessaire d'importer du plasma hyperimmunisé des Etats-Unis par exemple.
> Ainsi, cette pénurie d'immunoglobuline ne permet pas d'envisager une administration antenatale pour la prévention de la MHNN.

Divers travaux ont abouti à la production d'anticorps monoclonaux humains dans le but de remplacer les anticorps polyclonaux obtenus à partir du fractionnement de plasmas de donneurs volontaires.

Les anticorps monoclonaux présentent plusieurs avantages : ils peuvent être obtenus en grande quantité à des prix raisonnables, chaque lot d'anticorps est homogène et la qualité des différents lots est reproductible car ils sont produits par la même lignée cellulaire qui est cryopréservée dans l'azote liquide. La sécurité du produit peut être assurée quant à l'absence de contamination virale.

Plusieurs publications décrivent l'obtention de lignées cellulaires productrices d'anticorps monoclonaux humains anti-Rh D de classe IgG, à partir de cellules B de donneurs immunisés. Boylston et al. 1980 ; Koskimies 1980 ; Crawford et al. 1983 ; Doyle et al. 1985 ; Goossens et al. 1987 ; Kumpel et al. 1989(a) et Mc Cann-Carter et al. 1993 décrivent l'obtention de lignées de lymphocytes B transformés par le virus EBV. Melamed et al. 1985 ; Thompson et al. 1986 et Mc Cann-Carter et al. 1993 concernent des hétérohybrides résultant de la fusion lymphocytes B (transformés par EBV) x myélome murin. Goossens et al. 1987 porte sur des hétérohybrides résultant de la fusion lymphocytes B (transformés par EBV) x myélome humain. Bron et al. 1984 et Foung et al. 1987 décrivent des hétérohybrides résultant de la fusion lymphocytes B (transformés par EBV) x hétéromyélome homme-souris et enfin, Edelman et al. 1997 porte sur des cellules d'insectes transfectées avec le gène codant pour un anti-Rh(D) à l'aide du système baculovirus. Klein P. et al, Human antibodies, 1997, 8(1), 17-25 et Hadley A. et al Immunology, 1992, 76(5), 446-51 décrivent l'effet d'un changement de sous-classe IgG1/3 sur l'activite d'un anti-D

Parmi les brevets et demandes de brevet concernant de tels anticorps monoclonaux et leurs lignées sécrétrices, on peut citer :

> EP 576093 (AETS (FR), Biotest Pharma GmbH (Germany); Composition for prophylaxis of the haemolytic disease of the new-born comprises two human monoclonal antibodies of sub-class IgG1 and IgG3, which are active against the Rhesus D antigen), RU 2094462, WO 85/02413 (Board of Trustees of the Leland Stanford Jr. University, Human Monoclonal Antibody against Rh (D) Antigen and its Uses), GB 86-10106 (Central Blood Laboratories Authority, Production of heterohybridomas for manufacture of human monoclonal antibodies to Rhesus D antigen), EP 0 251 440 (Central Blood Laboratories Authority, Human Anti-Rhesus D Producing Heterohybridomas), WO 89/02442, WO 89/02600 et WO 89/024443 (Central Blood Laboratories Authority, Human Anti-Rh (D) Monoclonal Antibodies), WO 8607740 (Institut Pasteur, Protein Performance SA, Paris, FR, Obtention d'un anticorps monoclonal recombinant à partir d'un anticorps monoclonal human anti-rhésus D, sa production en cellules d'insecte et ses utilisations), JP 88-50710 (International Reagents Corp., Japan, Reagents for Determination of Blood Group Substance Rh (D) Factor), JP 83-248865 (Mitsubishi Chemical Industries Co., Ltd., Japan, Preparation of Monoclonal Antibody to Rh (D) positive Antigen), CA 82-406033 (Queens University at Kingston, Human Monoclonal Antibodies) et GB 8226513 (University College London, Human Monoclonal Antibody against Rhesus D Antigen).

Si l'utilisation d'anticorps monoclonaux présente de nombreux avantages par rapport à l'utilisation de pools d'anticorps polyclonaux, il peut en revanche s'avérer difficile d'obtenir un anticorps monoclonal efficace. En effet, on a trouvé dans le cadre de l'invention que le fragment Fcγ de l'immunoglobuline obtenue doit posséder des propriétés bien particulières afin de pouvoir interagir et activer les récepteurs des cellules effectrices (macrophage, lymphocyte T H et NK).

L'activité biologique de certaines immunoglobulines G est dépendante de la structure des oligosaccharides présents sur la molécule, et notamment sur sa partie Fc. Les molécules IgG de toutes les sous-classes humaines et murines possèdent un N-oligosaccharide fixé au domaine $CH_2$ de chaque chaîne lourde (au résidu Asn 297 pour les IgG humaines). L'influence de ce résidu glycannique sur la capacité de l'anticorps à interagir avec des molécules effectrices (Fc récepteurs et complément) a été démontrée. L'inhibition de glycosylation d'une IgG1 humaine, par culture en présence de Tunicamycine, provoque par exemple une diminution de 50 fois de l'affinité de cet anticorps pour le récepteur Fc$\gamma$RI présent sur les monocytes et macrophages (Leatherbarrow et al, 1985). La fixation au récepteur Fc$\gamma$RIII est également affectée par la perte de carbohydrates sur l'IgG, puisqu'il a été décrit qu'une IgG3 non glycosylée est incapable d'induire une lyse de type ADCC par l'intermédiaire du récepteur Fc$\gamma$RIII des cellules NK (Lund et al, 1990).

Mais, au-delà de la présence nécessaire de ces résidus glycanniques, c'est plus précisément l'hétérogénéité de leur structure qui peut aboutir à des différences dans la capacité à engager des fonctions effectrices. Des profils de galactosylation variables en fonction des individus (IgG1 humaines sériques) ont été observés. Ces différences reflètent probablement des disparités dans l'activité des galactosyltransférases et autres enzymes entre les clones cellulaires de ces individus (Jefferis et al, 1990). Alors que cette hétérogénéité normale des processus post-traductionnels génère différentes glycoformes (même dans le cas d'anticorps monoclonaux), elle peut conduire à des structures atypiques associées à certains états pathologiques comme l'arthrite rhumatoïde, la maladie de Crohn, pour lesquelles une proportion importante de résidus agalactosylés a été mise en évidence (Parekh et al, 1985).

Le profil de glycosylation de la molécule purifiée est la conséquence d'effets multiples dont certains paramètres ont déjà été étudiés. Le squelette protéique des IgG et notamment les acides aminés en contact avec les résidus N-acétylglucosamine (GlcNAc) et galactose terminaux du bras mannose $\alpha$ 1-6 (aa 246 et 258 des IgG) peuvent expliquer l'existence de structures préférentielles (galactosylation) comme le montre l'étude réalisée sur les IgG murines et chimériques de différents isotypes (Lund et al, 1993).

Les différences observées mettent également en évidence des spécificités liées à l'espèce et au type cellulaire utilisés pour la production de la molécule. Ainsi, la structure classique des N-glycannes des IgG humaines révèle une proportion significative de types biantennés avec un résidu GlcNAc en position bissectrice, structure absente au niveau des anticorps produits par des cellules murines. De même, les résidus d'acides sialiques synthétisés par la lignée CHO (Chinese Hamster Ovary) sont exclusivement de type $\alpha$ 2-3 alors qu'ils sont de type $\alpha$ 2-3 et $\alpha$ 2-6 avec les cellules murines et humaines (Yu Ip et al, 1994). La production d'immunoglobulines dans des systèmes d'expression autres que ceux issus de mammiferes peut introduire des modifications beaucoup plus importantes comme la présence de résidus xylose fabriqués par les cellules d'insectes ou par les plantes (Ma et al, 1995).

D'autres facteurs comme les conditions de culture cellulaire (incluant la composition du milieu de culture, la densité cellulaire, le pH, l'oxygénation) semblent intervenir sur l'activité des glycosyltransférases de la cellule et par conséquent sur la structure glycannique de la molécule (Monica et al, 1993; Kumpel et al, 1994 b).

Or, on décrit ci-après qu'une structure de type biantennée, avec des chaînes courtes, une faible sialylation, des mannoses terminaux et/ou GlcNAc terminaux non intercalaires est le dénominateur commun des structures glycanniques conférant une forte activité ADCC aux anticorps monoclonaux. On a mis au point un procédé de préparation de tels anticorps capables d'activer les cellules effectrices par l'intermédiaire du Fc$\gamma$RIII, notamment d'anticorps anti-Rh(D).

Les antigènes de groupes sanguins sont classés en plusieurs systèmes en fonction de la nature des molécules membranaires exprimées à la surface des hématies. Le système Rhésus (Rh) comprend 5 molécules ou antigènes : D, C, c, E et e (ISSITT, 1988). L'antigène D est la plus importante de ces molécules parce qu'elle est la plus immunogène, c'est-à-dire qu'elle peut induire la production d'anticorps anti-D si des globules rouges Rh D positif sont transfusés à des sujets Rh négatif.

L'antigène D est normalement exprimé chez 85 % des sujets caucasoïdes, ces personnes sont dites Rh positif ; 25 % des ces sujets sont donc Rh négatif c'est-à-dire que leurs hématies ne présentent pas d'antigène D. L'expression des antigènes D présente certaines variantes qui peuvent être liées soit à une faible densité antigénique, on parlera alors d'antigènes D faibles, soit à une antigénicité différente ou partielle, on parlera alors d'antigènes D partiels. Le caractère D faible est caractérisé par le fait qu'il s'agit d'un antigène normal mais dont le nombre de sites par hématie est diminué de manière plus ou moins importante ; ce caractère est transmissible selon les lois mendéliennes. Les phénotypes D partiels ont été découverts chez des sujets Rh D positif qui possédaient des anticorps anti-D sériques ; ces antigènes D partiels peuvent donc être caractérisés comme ne possédant qu'une partie de la mosaïque. Des études réalisées avec des anticorps polyclonaux et monoclonaux ont permis de définir 7 catégories d'antigènes D partiels avec la description d'au moins 8 épitopes constitutifs de l'antigène D (LOMAS et al. 1989 ; TIPETT 1988).

L'importance des anticorps anti-Rh D est apparue avec la découverte des mécanismes entraînant la maladie hémolytique du nouveau-né (MHNN). Celle-ci correspond aux différents états pathologiques observés chez certains foetus ou chez certains nouveau-nés lorsqu'il y a une incompatibilité foeto-maternelle de groupe sanguin qui est responsable de la formation d'anticorps maternels anti-Rh D capables de traverser la barrière placentaire. En effet, le passage d'hématies foetales Rh positif chez une mère Rh négatif peut entraîner la formation d'anticorps anti-D.

Après immunisation de la mère Rh négatif, les anticorps anti-D de classe IgG sont capables de traverser la barrière placentaire et de se fixer sur les hématies foetales Rh positif. Cette fixation entraîne l'activation de cellules immunocompétentes via leurs récepteurs Fc de surface induisant ainsi une hémolyse des hématies foetales sensibilisées. En fonction de l'intensité de la réaction, plusieurs degrés de gravité de la MHNN peuvent être observés.
Un diagnostic de la MHNN peut être réalisé avant et après la naissance. Le diagnostic prénatal est basé sur l'évolution du taux des anticorps anti-D chez la mère en utilisant plusieurs techniques immunohématologiques. Le diagnostic post-partum peut être fait à partir d'un prélèvement de sang de cordon en analysant les paramètres suivants : détermination des groupes sanguins du foetus et du père ; recherche d'anticorps anti-D ; dosages de l'hémoglobine et de la bilirubine.
Une prophylaxie de la MHNN est actuellement systématiquement réalisée chez toutes les femmes de groupe sanguin Rh négatif ayant mis au monde un enfant Rh positif avec des injections d'immunoglobulines humaines anti-D. Les premiers essais réels d'immunoprophylaxie ont débuté en 1964. Pour que la prévention soit efficace, il faut que les immunoglobulines soient injectées avant l'immunisation c'est-à-dire dans les 72 heures qui suivent l'accouchement et que les doses d'anticorps soient suffisantes (10 $\mu$g d'anticorps anti-D pour 0,5 ml d'hématies Rh+).
Plusieurs anticorps monoclonaux anti-D ont fait l'objet d'une évaluation thérapeutique : BROSSARD/FNTS 1990 (non publié) ; THOMSON/IBGRL 1990 ; KUMPEL/IBGRL 1994 ; BELKINA/Institut d'hématologie Moscou 1996 ; BIOTEST/LFB 1997 (non publié). L'efficacité clinique des anticorps à induire la clairance de globules rouges Rh(D) positif a été évaluée chez des volontaires Rh(D) négatif.
Un seul anticorps de type IgG1 a montré une efficacité équivalente à celle des immunoglobulines polyclonales anti-D mais seulement sur quelques patients (KUMPEL et al, 1995).
Des anticorps sélectionnés par un test du type ADCC spécifique du et/ou des anticorps possédant une structure glycannique nécessaire à l'obtention d'une bonne efficacité sont décrits.

**Description**

Ainsi, la présente invention se rapporte à un procédé de préparation d'un anticorps monoclonal capable d'activer les cellules effectrices exprimant le FcγRIII caractérisé en ce qu'il comprend les étapes suivantes :

a) purification d'anticorps monoclonaux obtenus à partir de différents clones provenant de lignées cellulaires sélectionnées parmi les lignées cellulaires hétérohybrides obtenues par fusion de lymphocytes B humains provenant de sujets immunisés avec des cellules du myélome K6H6-B5 (ATCC n°CRL 1823) et la lignée cellulaire YB2/0 transfectée à l'aide d'un vecteur contenant le gène codant pour une immunoglobuline humaine de type IgG;
b) addition de chaque anticorps obtenu à l'étape a) dans un mélange réactionnel distinct comprenant :

- les cellules cibles desdits anticorps,
- des cellules effectrices comprenant des cellules exprimant le FcγRIII,
- des IgG polyvalentes humaines thérapeutiques,

c) détermination du pourcentage de lyse des cellules cibles et sélection des anticorps monoclonaux qui activent les cellules effectrices provoquant une lyse significative des cellules cibles (activité ADCC de type FcγRIII), caractérisé en ce que les récepteurs FcγRIII sont saturés par l'addition d'IgG polyvalentes humaines thérapeutiques.

Les IgG polyvalentes sont utilisées pour inhiber le mécanisme de lyse des cellules effectrices via le FcγRIII. Dans ce procédé, on sélectionne les anticorps présentant un taux ADCC de type FcγRIII supérieur à 60 %, 70%, 80 % ou de préférence supérieur à 90 %.
Les cellules cibles peuvent être des hématies traitées à la papaïne. Dans ce cas, on dépose par puits :

- 100$\mu$l d'anticorps monoclonaux purifiés à environ 200 ng/ml,
- 25$\mu$l d'hématies papaïnées, soit environ 1x$10^6$ cellules,
- 25$\mu$l de cellules effectrices, soit environ 2x$10^6$ cellules,
- et 50$\mu$l d'IgG polyvalentes, notamment de TEGELINE™ (LFB, France), à une concentration comprise entre 1 et 20 mg/ml.

On peut ainsi comparer la quantité de lyse des cellules cibles à deux témoins positifs consistant en un composé chimique tel que du $NH_4Cl$ et un anticorps de référence actif *in vivo* et à un témoin négatif consistant en un anticorps inactif *in vivo*.
On peut également utiliser les anticorps polyclonaux d'origine commerciale comme témoins positifs et un anticorps monoclonal inapte à induire une clairance *in vivo* comme témoin négatif.
Avantageusement, ce procédé permet de préparer des anticorps monoclonaux anti-Rh(D), comme indiqué

précédemment. On utilise alors comme cellules cibles des hématies rhésus D.

L'invention repose donc sur la mise au point d'un test d'activité biologique *in vitro* dans lequel les activités mesurées sont en corrélation avec l'activité biologique *in vivo* des anticorps monoclonaux ou polyclonaux déjà évalués du point de vue clinique quant à leur potentialité à induire la clairance d'hématies Rh (D) positives chez des sujets volontaires Rh (D) négatifs. Ce test permet d'évaluer l'activité lytique dépendante de l'anticorps = ADCC (Antibody Dependent Cellular Cytotoxicity) induite essentiellement par les récepteurs Fcγ de type III (CD 16), les récepteurs Fcγ de type I (CD64) étant saturés par l'addition d'immunoglobulines humaines de type IgG (sous la forme d'IgG polyvalentes thérapeutiques). La spécificité FcγRIII de ce test ADCC a été confirmée par inhibition en présence d'anticorps monoclonal anti-FcγRIII (voir figure 6). Des cellules mononucléées de sujets sains sont utilisées comme cellules effectrices dans un ratio effecteur/cible (E/C) proche des conditions physiologiques *in vivo*. Dans ces conditions les activités lytiques des immunoglobulines polyclonales et des anticorps monoclonaux anti-D inefficaces *in vivo* (anticorps DF5 Goossens et al, 1987 et les anticorps AD1+AD3, FR 9207893 LFB/Biotest et FOG-1, GB 2189506) sont respectivement fortes et faibles.

La sélection des anticorps décrits a donc été réalisée par évaluation de leur activité biologique dans ce test de type ADCC (voir exemple 1).

Sont également décrits des anticorps susceptibles d'être obtenus à partir du procédé décrit ci-dessus, lesdits anticorps présentant des taux ADCC de type FcγRIII supérieurs à 60 %, 70%, 80 % ou de préférence supérieur à 90 % par rapport au polyclonal de référence. Ces anticorps monoclonaux, dirigés contre un antigène donné, activent les cellules effectrices exprimant le FcγRIII provoquant une lyse supérieure à 60 %, 70 %, 80 %, de préférence supérieure à 90 % de la lyse provoquée par des anticorps polyclonaux dirigés contre ledit antigène. Lesdits anticorps monoclonaux peuvent être dirigés contre le rhésus D.

Ils peuvent être produit par des clones dérivés des lignées Vero (ATCC n° CCL 81), YB2/0 (ATCC n° CRL 1662) ou CHO Lec-1 (ATCC n° CRL 1735) et peuvent appartenir à la classe IgG1 ou IgG3.

Sont également décrits des anticorps ayant une structure glycannique particulière conférant une activité effectrice dépendante du FcγRIII.

De tels anticorps peuvent être obtenus à partir d'un procédé explicité ci-dessus et possèdent sur leur site de glycosylation (Asn 297) du Fcγ des structures glycanniques de type biantennées, avec des chaînes courtes et une faible sialylation. De préférence, leur structure glycannique présente des mannoses terminaux et/ou GlcNAc terminaux non intercalaires.

De tels anticorps sont plus particulièrement sélectionnés parmi les formes :

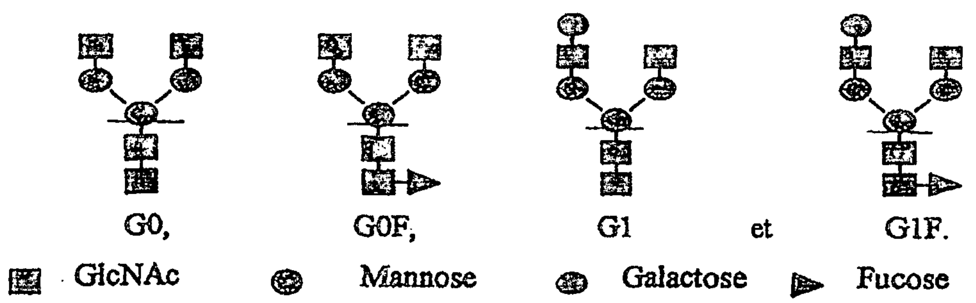


Ainsi, il est décrit un anticorps monoclonal possédant sur son site de glycosylation (Asn 297) du Fcγ des structures glycanniques de type biantennées, avec des chaînes courtes, une faible sialylation, des mannoses et GlcNAc du point d'attache terminaux non intercalaires. Lesdits anticorps, dirigés contre un antigène donné, activent les cellules effectrices exprimant le FcγRIII provoquant une lyse supérieure à 60 %, 70 %, 80 %, de préférence supérieure à 90 % de la lyse provoquée par des anticorps polyclonaux dirigés contre ledit antigène.

Plus particulièrement, des anticorps et des compositions comprenant lesdits anticorps tels que définis ci-dessus, ont une teneur en acide sialique inférieure à 25%, 20%, 15%, ou 10%, de préférence 5%, 4% 3% ou 2%.

De même, des anticorps et des compositions comprenant lesdits anticorps tels que définis ci-dessus, présentent une teneur en fucose inférieure à 65%, 60%, 50%, 40%, ou 30%. De préférence, la teneur en fucose est comprise entre 20% et 45% ou encore entre 25% et 40%.

Une composition particulièrement efficace comprend par exemple une teneur supérieure à 60%, de préférence supérieure à 80% pour les formes G0 + G1 + G0F + G1F étant entendu que les formes G0F + G1F sont inférieure à 50%, de préférence inférieure à 30%.

**Tableau 1 : quantification (%) des structures oligosaccharidiques des différents anticorps Anti-RhD**

| | *Anticorps actifs en ADCC FcRγIII* | | | | | *Anticorps inactifs en ADCC FcRγIII* | | |
|---|---|---|---|---|---|---|---|---|
| | R 297 | R 270 | | F60 | | D 31 | | F5 |
| Structure | HPCE-LIF | HPCE-LIF | HPLCs | HPCE-LIF | HPLCs | HPCE-LIF | HPCE-LIF | HPLCs |
| *Fucosylé* | 34,3 | 45,9 | 37,2 | 47,7 | 46,6 | 82,0 | 88 | 100 |
| *Sialylé* | 1,0 | 2,2 | 4,1 | 9,9 | 19,6 | 47,9 | 52,0 | 47 |
| G2S2FB | | | | | | | | 2,8 |
| G2S2F | 0,0 | 0,0 | n.d. | 4,2 | 0,0 | 11,3 | 11,9 | 4,1 |
| G2S1FB | | | | | | | | 6,1 |
| G2S1F | 1,0 | 1,0 | n.d. | 2,7 | 2,5 | 21,4 | 30,5 | 28 |
| G2S1 | 0,0 | 1,2 | n.d. | 3,0 | 0,0 | 0 | 0 | |
| G1S1FB | | | | | | | | 6,2 |
| G1S1F | | | | | | | | 1,7 |
| G2F | 3,9 | 5,0 | 3,0 | 10,3 | 11,6 | 16,9 | 22,1 | 4,2 |
| G2 | 12,1 | 6,1 | 3,3 | 7,0 | 13,3 | 2,0 | 0,0 | 0,0 |
| G1FB | | | | | | | | 25,7 |
| G1F | 17,4 | 16,9 | 15 | 24,8 | 22,1 | 16,1 | 21,5 | 12,4 |
| G1 | 26,1 | 11,3 | 21,0 | 22,2 | 22,8 | 0,0 | 0,0 | 0,0 |
| G0F | 12,1 | 23,1 | 19,4 | 5,6 | 10,5 | 1,7 | 3,0 | 0,0 |
| G0 | 29,1 | 32,7 | 38,5 | 15,8 | 17,7 | 13,6 | 13,9 | 0,5 |

Une alternative pour cibler spécifiquement le FcγRIII consiste en la préparation d'anticorps du type « high mannose ».

Dans un autre aspect, une cellule produisant un anticorps mentionné ci-dessus est décrite. Il peut s'agir d'un hybridome, notamment d'un hétérohybridome obtenu avec le partenaire de fusion K6H6-B5 (ATCC n°CRL 1823) ; ou d'une cellule animale ou humaine transfectée à l'aide d'un vecteur comportant le gène codant pour ledit anticorps, notamment une cellule dérivée des lignées Vero (ATCC n° CCL 81), YB2/0 (ATCC n° CRL 1662) ou CHO Lec-1 (ATCC n° CRL 1735). Ces cellules correspondent aux lignées cellulaires sélectionnés par le procédé selon l'invention, lesdites cellules produisant des anticorps possédant les caractéristiques évoquées précédemment.

Un anticorps peut montrer une activité biologique importante (supérieure ou égale à celle de l'anticorps polyclonal anti-Rh(D) de référence) dans le test ADCC utilisant des cellules effectrices FcγRIII positives.
Son aptitude à l'activation des récepteurs FcγRIII (après fixation) est confirmée sur des modèles *in vitro* qui mettent en évidence la modification du flux calcique intracellulaire, la phosphorylation de molécules de transduction du signal d'activation ou la libération de médiateurs chimiques.
Ces propriétés sont associées à une stucture particulière des oligosaccharides du site de N-glycosylation de la partie Fc de l'anticorps : présence de chaînes courtes, faiblement galactosylées, peu sialylées, possédant des mannoses terminaux et/ou GlcNAc terminaux non intercalaires par exemple.
Cet anticorps présente des applications thérapeutiques : prévention de la MHNN, traitement du PTI chez les individus Rh(D) positif, et toute autre application concernée par l'utilisation d'immunoglobulines polyclonales anti-D.
Un anticorps peut également avoir une spécificité autre que anti-Rh(D) (anti-cellule cancéreuse par exemple). Il peut posséder les propriétés décrites précédemment (activité fonctionnelle dépendante d'un mécanisme de fixation/activation au niveau des récepteurs FcγRIII, structure particulière des oligosaccharides) et être utilisé dans l'immunothérapie de cancers ou de toute autre pathologie pour laquelle peut être effectué un traitement curatif ou préventif à l'aide d'un anticorps monoclonal dont le mécanisme d'action correspond à une activité fonctionnelle par l'intermédiaire du récepteur

FcγRIII.

Un autre aspect porte sur une composition pharmaceutique comprenant un anticorps décrit ci-dessus et sur l'utilisation dudit anticorps pour la fabrication d'un médicament.

Un anticorps anti-Rh(D) décrit ci-dessus peut être utilisé pour la fabrication d'un médicament destiné à la prévention de l'allo-immunisation Rhésus d'individus Rh négatif. Le mode d'action des immunoglobulines anti-D *in vivo* est une fixation spécifique des anticorps sur l'antigène D des globules rouges Rh(D) positif, suivie d'une élimination de ces globules rouges de la circulation essentiellement au niveau de la rate. Cette clairance est associée à un mécanisme dynamique de suppression de la réponse immune primaire chez les individus et prévient donc l'immunisation.

Ainsi, on peut utiliser un tel anticorps de manière prophylactique pour la prévention de l'alloimmunisation de femme Rhésus négatif, immédiatement après la naissance d'un enfant Rhésus positif, et pour prévenir, lors des grossesses ultérieures, la maladie hémolytique du nouveau-né (MHNN) ; lors d'avortements, de grossesses extra-utérines en situation d'incompatibilité Rhésus D ou encore lors d'hémorragies transplacentaires résultant d'amniocentèse, de biopsies chorioniques, ou de manipulations obstétriques traumatisantes en situation d'incompatibilité Rhésus D.

En outre, on peut utiliser un tel anticorps dans le cas de transfusions Rh incompatibles avec du sang ou des dérivés sanguins labiles.

Un anticorps ci-dessus décrit peut être utilisé pour la fabrication d'un médicament destiné à une utilisation thérapeutique dans le Purpura Thrombocytopénique Idiopathique (PTI).

Les anticorps décrits sont également utiles pour la fabrication d'un médicament destiné au traitement de cancers par immunothérapie ou pour le traitement d'infections causées par des agents pathogènes viraux ou bactériens.

Un aspect complémentaire porte sur l'utilisation desdits anticorps notamment pour le diagnostic et concerne donc un kit comprenant un anticorps décrit ci-dessus.

Pour la suite de la description, on se référera aux légendes des figures présentées ci-après.

**Légendes**

**Figure 1** : **évaluation ADCC de F60 et T125 YB2/0 (R270)**

Cette figure représente le pourcentage de lyse obtenue en fonction de la concentration en anticorps en présence de 100 et 500 μg/puits de TEGELINE™ (LFB, France). On obtient un pourcentage de lyse élevé pour les anticorps selon l'invention F60 et T125.

**Figure 2** : **fixation des anti-D au récepteur (FcγRIII)**

On obtient un fort indice de fixation pour les anticorps selon l'invention F60 et T125.

**Figure 3** : **construction du vecteur d'expression T125-H26 pour l'expression de la chaîne H de T125.**

**Figure 4 : construction du vecteur d'expression T125-K47 pour l'expression de la chaîne L de T125**

**Figure 5 : construction du vecteur d'expression T125-IG24 pour l'expression de l'anticorps entier T125.**

**Figure 6 : inhibition ADCC en présence d'anti Fc RIII (CD16)**

Le test ADCC est établi selon le mode opératoire décrit au §3.3 en présence de l'anti-CD16 commercial 3G8 (TEBU) dont l'action est de bloquer les récepteurs FcRIII présents sur les cellules effectrices. La concentration finale de 3G8 est de 5μg/puits (25μg/ml). Un témoin est fait en parallèle en absence de 3G8.

Les trois anticorps testés sont le Poly-D WinRho, l'anticorps F60 (Pf 155 99/47)obtenu selon le procédé décrit en exemple 1 et le R297 (Pf 210 01/76) obtenu selon le procédé décrit en exemple II.

Résultats : on observe une inhibition en présence du 3G8, ce qui démontre que l'ADCC induite par les trois anticorps testés est majoritairement dépendante du FcRIII.

Une inhibition légèrement plus forte est observée en présence du Poly-D WinRho (83% par rapport à 68% et 61 % d'inhibition pour le F60 et R297 respectivement). Cette différence peut être due à la présence dans le Poly-D d'IgG humaines non anti-D qui vont inhiber les récepteurs de type 1 (FCRI ou CD64) et donc agir en synergie avec l'anti-CD16.

**Figure 7** : **caractérisation des glycanes des Anti-D par Spectrométrie de Masse (MS).**

**Figure 8** : **Comparaison des spectres MS de R 290 et DF5.**

**Figure 9** : **étude de la glycosylation de l'Anti-D D31DMM par MS.**

**EXEMPLE 1 : ETABLISSEMENT D'UNE LIGNEE CELLULAIRE HETEROHYBRIDE PRODUCTRICE D'UN ANTICORPS ANTI- RH (D)**

**1- Obtention de clones lymphoblastoïdes et d'hétérohybrides :**

***1.1- Source de lymphocytes :***

Le donneur de lymphocytes B est sélectionné parmi les donneurs d'anti-Rh(D) en plasmaphérèse, sur l'activité de ses anticorps anti-Rh(D) sériques dans le test d'activité ADCC décrit au §3.3. Après un don de sang total en 1998, la fraction «buffy coat» (concentré de leucocytes) est récupérée.

***1.2-Immortalisation des lymphocytes B du donneur***

Les cellules mononucléées du sang periphérique sont séparées des autres éléments par centrifugation sur Ficoll Plus (Pharmacia). Elles sont ensuite diluées à $10^6$ cellules/ml en IMDM contenant 20% (v/v) de sérum de veau foetal (SVF), auquel sont ajoutés 20% de surnageant de culture de la lignée B95-8 (ATCC-CRL1612), 0,1$\mu$g/ml de cyclosporine A (Sandoz), 50 $\mu$g/ml de sulfate de gentamycine (Life Technologies), et réparties en plaques de 24 puits (P24 Greiner) ou en plaques de 96 puits à fond rond. Elle sont ensuite placées en incubateur à 37°C, 7% CO2. Après 3 semaines, la présence d'anticorps anti-Rh(D) est recherchée par ADCC

^ Chacun des 16 micropuits d'un puits de plaque P24 positif est transféré dans un nouveau puits de P24. Cet enrichissement est répété après 10 à 15 jours de culture et chaque micropuits est amplifié en P96 puis en P24.
^ Les puits de P96 positifs sont repris et amplifiés en P24 à fond plat (Nunc). Après quelques jours de culture, la présence d'anticorps anti-Rh(D) est recherchée par ADCC.

***1.3- Enrichissement par rosettes immunes (RI) :***

Les cellules issues d'un ou plusieurs puits de P24 sont enrichies en cellules spécifiques par formation et séparation de rosettes avec des hématies Rh(D) positives papaïnées : 1 volume d'hématies lavées en NaCl 0,9% est incubé 10 minutes à 37°C avec 1 volume de solution de papaïne (Merck) au 1/1000$^{ème}$ (m/v), puis lavé 3 fois en NaCl 0,9 %. Les cellules sont ensuite lavées une fois en solution de Hanks, mises en suspension en SVF et mélangées aux hématies papaïnées dans le rapport 1 cellule pour 33 hématies. Le mélange est placé dans un tube à centrifuger à fond conique, centrifugé 5 minutes à 80g et incubé une heure dans la glace fondante. Le mélange est ensuite délicatement agité et le Ficoll déposé au fond du tube pour séparation 20 minutes à 900g. Le culot contenant les rosettes est hémolysé en solution de $NH_4Cl$ pendant 5 minutes et les cellules remises en culture en P24 contenant des cellules mononucléées humaines irradiées. Après environ 1 semaine, les surnageants sont évalués en tests CELA (paragraphe 3.2) et ADCC pour la présence d'anticorps anti-Rh(D) ayant une bonne activité. Un nouveau cycle d'enrichissement est réalisé si le pourcentage de cellules formant rosettes augmente significativement par rapport au cycle précédent.

***1.4-Clonages des cellules lymphoblastoïdes :***

Les cellules enrichies par RI sont réparties à 5 et 0,5 cellules par puits dans des plaques de 96 puits à fond rond contenant des cellules mononucléées humaines irradiées.

Après environ 4 semaines de culture, les surnageants des puits contenant des amas cellulaires sont évalués par test ADCC.

***1.5- Hétérofusion :***

Les puits de clonages des cellules transformées par EBV présentant une activité ADCC intéressante sont amplifiés en culture puis fusionnés avec l'hétéromyelome K6H6-B5 (ATCC CRL1823) selon la technique standard au PEG. Après fusion, les cellules sont réparties à raison de 2 $10^4$ cellules/puits en P96 à fond plat contenant des macrophages intrapéritonéaux murins et dans un milieu sélectif contenant de l'aminoptérine et de la ouabaïne (Sigma).
Après 3 à 4 semaines de culture, les surnageants des puits contenant des amas cellulaires sont évalués par test ADCC.

***1.6- clonages des hétérohybridomes :***

Le clonage par dilution limite est effectué à 4, 2 et 1 cellules/puits dans des P96 à fond plat. Après 2 semaines, l'aspect microscopique des puits est examiné pour identifier les clones uniques puis le milieu est renouvelé. Au bout de

2 semaines environ, les surnageants des puits contenant des amas cellulaires sont évalués par test ADCC.

**2- Historique des clones retenus :**

***2.1- clone producteur d'une IgG1***

La transformation par EBV des cellules du donneur d13 a permis la sélection d'un puits, désigné T125 2A2 sur lequel ont été réalisés successivement : 2 enrichissements, 3 cycles de RI, et un clonage à 5 cellules/puits pour donner 2 clones :

1) T125 2A2 (5/1)A2 à partir duquel a été extrait l'ADN pour la préparation du vecteur recombinant ;
2) T125 (5/1)A2 qui a été fusionné avec K6H6-B5 pour donner F60 2F6 puis après 5 clonages F60 2F6 (5) 4C4, clone retenu pour la constitution d'un stock cellulaire préalable à la préparation de banques.

Il s'agit d'une IgG1 possédant une chaîne légère Kappa.

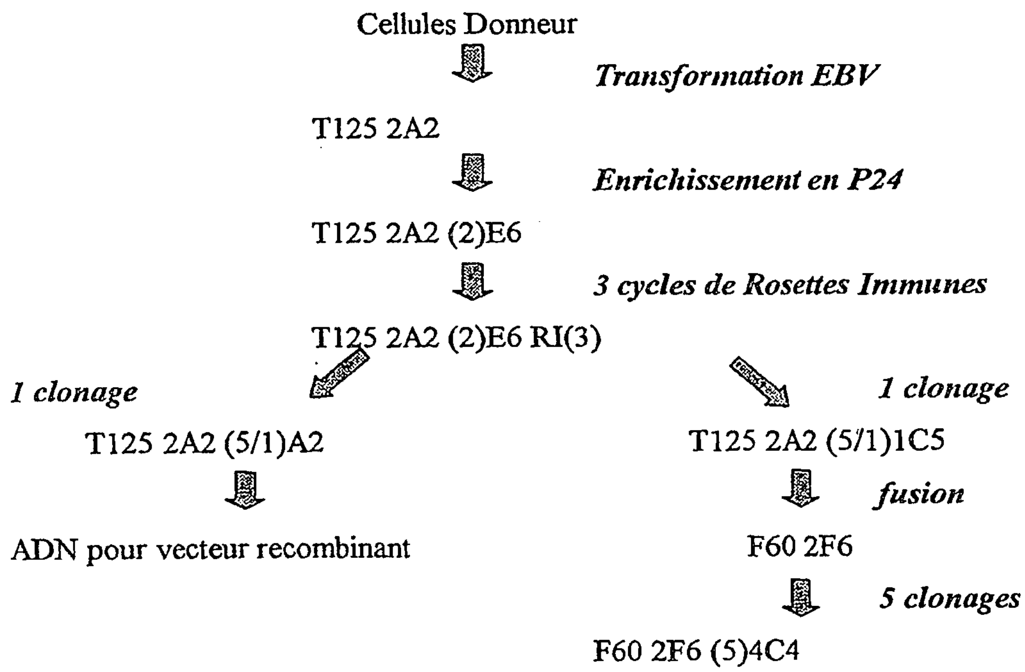


***2.2- clone producteur d'une IgG3***

Selon le même procédé que celui utilisé pour la préparation de l'anticorps d'isotype IgG1, une lignée productrice d'une IgG3 a été préparée. Les cellules d'origine proviennent d'un don de sang total, d'un autre donneur désigné, dont la fraction « buffy coat » (concentré de leucocytes) a été récupérée.
Il s'agit d'une IgG3 possédant une chaîne légère Kappa.

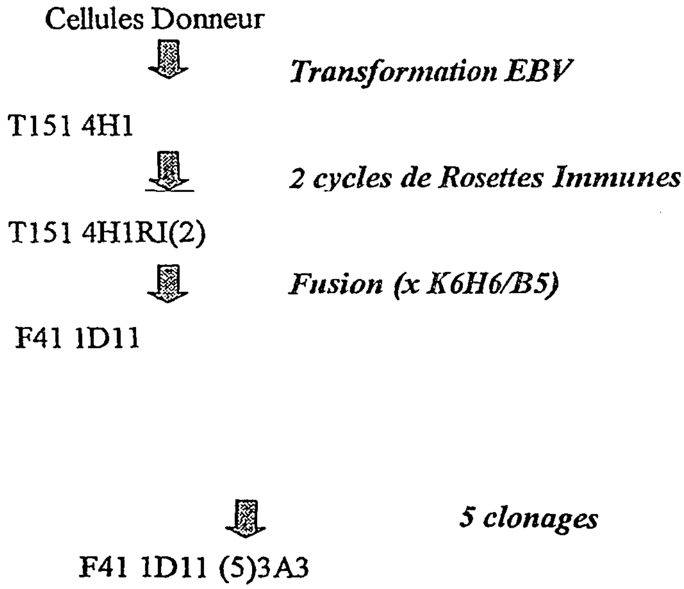


### 3- Méthodes d'évaluation des anticorps anti-Rh (D) :

Après purification par chromatographie d'affinité sur protéine A Sépharose (Pharmacia) et dialyse en tampon Tris 25mM, NaCl 150mM, pH 7,4 , la concentration de l'anticorps T125 est déterminée par technique ELISA. L'activité biologique in vitro est ensuite mesurée par la technique ADCC.

***3.1- Détermination du taux d'IgG et des isotypes par technique ELISA*** **:**

- *IgG totales*
  Coating : anti-IgG (Calbiochem) à 2μg/ml en tampon carbonate 0,05M pH 9.5, 1 nuit à 4°C. Saturation : tampon de dilution (PBS + 1% BSA+ 0,05% Tween 20, pH 7.2) 1h à température ambiante. Lavage (à renouveler à chaque étape) : H2O + NaCl 150mM + 0,05% Tween 20. Dilution des échantillons en tampon de dilution à environ 100ng/ml et de la gamme témoin constituée à partir d'IgG humaine polyvalentes LFB prédiluées à 100ng/ml. Incubation 2h à température ambiante. Conjugué : anti-IgG (Diagnostic Pasteur) dilué au 1/5000, 2heures à température ambiante. Substrat : OPD à 0,5mg/ml (sigma) en tampon phosphate citrate, perborate de Na (Sigma), 10 minutes à l'oscurité. Arrêt de la réaction par HCL 1N, et lecture à 492 nm.

- *Dosage chaine Kappa.*
  Coating : anti-Kappa (Caltag Lab) à 5μg/ml en tampon carbonate 0,05M pH 9.5,1 nuit à 4°C. Saturation : tampon de dilution (PBS + 1% BSA+ 0,05% Tween 20, pH 7.2) 1h à température ambiante. Lavage (à renouveler à chaque étape) : H2O + NaCl 150mM + 0,05% Tween 20. Dilution des échantillons en tampon de dilution à environ 100ng/ml et de la gamme témoin constituée à partir de l'anticorps monoclonal AD3T1 LFB (Kappa/gamma 3) prédilué à 100ng/ml. Incubation 2 h à température ambiante Conjugué : anti-kappa biotinylé (Pierce) dilué au 1/1000 en présence de Streptavidine-peroxidase (Pierce) dilué au 1/1500, 2 heures à température ambiante. Substrat : OPD à 0,5mg/ml (sigma) en tampon phosphate citrate, perborate de Na (Sigma), 10 minutes à l'obscurité. Arrêt de la réaction par HCL 1N, et lecture à 492 nm.

***3.2- Dosage spécifique anti-D par technique CELA (Cellular Enzyme Linked Assay):***

Cette méthode est utilisée pour le dosage spécifique des anticorps anti-D notamment lorsqu'il s'agit de surnageant de culture à des stades de culture où d'autres immunoglobulines non anti-D sont présentes dans la solution (étapes précoces après transformation EBV).
Principe : l'anticorps anti-D est incubé avec des hématies Rhésus positif puis révélé par une anti-Ig humaine marquée à la phosphatase alcaline.
100μl d'hématies Rh+ à 10% diluées en tampon de dilution Liss-BSA 1%. Dilution des échantillons en tampon de dilution à environ 500ng/ml et de la gamme témoin constituée à partir d'une IgG anti-D humaine monoclonale purifiée (DF5,

LFB) prédiluée à 500ng/ml. Incubation 45 min à température ambiante. Lavage (à renouveler à chaque étape) : H2O + NaCl 150mM. Conjugué : anti-IgG phosphatase alcaline (Jackson) dilué au 1/4000 en PBS+1% BSA, 1h30 à température ambiante. Substrat : PNPP à 1mg/ml (sigma) en diéthanolamine 1M, MgCl2 0,5mM ; PH 9,8. Arrêt de la réaction par NaOH 1N et lecture à 405 nm.

### *3.3- Technique ADCC*

La technique ADCC (Antibody-Dependent Cellular Cytotoxicity) permet d'évaluer la capacité des anticorps (anti-D) à induire la lyse des hématies Rh positif, en présence de cellules effectrices (cellules mononucléées ou lymphocytes).
Brièvement, les hématies d'un concentré globulaire Rh positif sont traitées à la papaïne (1 mg/ml, 10 min à 37°C) puis lavées en NaCL 0,9%. Les cellules effectrices sont isolées à partir d'un pool d'au moins 3 buffy-coat, par centrifugation sur Ficoll (Pharmacia), suivi d'une étape d'adhérence en présence de 25% de SVF, de façon à obtenir un ratio lymphocytes/monocytes de l'ordre de 9. Dans une plaque de microtitration (96 puits) on dépose par puits : 100μl d'anticorps anti-D purifié à 200 ng/ml, 25μl d'hématies papaïnées Rh+ (soit $1x10^6$), 25μl de cellules effectrices (soit $2x10^6$) et 50μl d'IgG polyvalentes (Tégéline LFB par exemple) aux concentrations usuelles de 10 et 2 mg/ml. Les dilutions sont faites en IMDM 0,25% SVF. Après incubation 1nuit à 37°C, les plaques sont centrifugées, puis l'hémoglobine libérée dans le surnageant est mesurée en présence d'un substrat spécifique de l'activité peroxydasique (2,7 diaminofluorène, DAF). Les résultats sont exprimés en pourcentage de lyse, 100% correspondant à la lyse totale des hématies en NH4Cl (témoin 100%), et 0% au mélange réactionnel sans anticorps (témoin 0%).
La lyse spécifique est calculée en pourcentage selon la formule suivante :

$$\frac{(\text{DO échantillon} - \text{DO témoin } 0\%) \times 100}{\text{DO témoin } 100\% - \text{DO témoin } 0\%} = \%\ \text{ADCC}$$

Les résultats présentés à la figure 1 montrent l'activité de l'anticorps produit par l'hétérohybride F60 comparée à celles des anticorps de référence :

- les anticorps polyclonaux anti-Rh(D) POLY-D LFB 51 et WinRhO W03 (Cangene) = témoins positifs
- l'anticorps monoclonal DF5 (inactif *in vivo* sur la clairance d'hématies Rh(D) positives (BROSSARD/FNTS, 1990, non publié)) = témoin négatif
- les IgG1 purifiées (séparées des IgG3) à partir du polyclonal WinRhO W03.

Deux concentrations d'IgG humaines (Tégéline LFB) sont utilisées pour montrer que l'inhibition d'activité du témoin négatif est liée à la fixation des IgG compétitrices sur les récepteurs Fcγ de type I.

### *3.4- Technique de fixation au FcγRIII(CD16) :*

Ce test permet d'apprécier la fixation des anticorps anti-Rh(D) d'isotype IgG1 sur le FcγRIII et notamment de différencier des anticorps IgG3. Compte tenu de la faible affinité de ce récepteur pour les IgG monomériques, la fixation préalable des anticorps sur l'antigène D est nécessaire.
Principe : sur des membranes d'hématies Rh+ coatées en plaque de microtitration, on ajoute l'anticorps à tester (anti-D) puis des cellules Jurkat transfectées exprimant à leur surface le récepteur FcγRIII. Après centrifugation, l'interaction « membrane Rh+/anti-D/Jurkat CD16 » se visualise par un étalement homogène des Jurkat CD16 dans le puits. Au contraire, les cellules se regroupent au centre du puits en absence d'interaction. L'intensité de la réaction est exprimée en nombres de +.
Méthode :

1) Incubation 1h à 37°C de l'anticorps anti-D (50 μl à 1μg/ml en IMDM) sur plaque de Capture R (Immunochim), puis lavages en eau + NaCl 0,9%. Ajout de Jurkat CD16 ($2.10^6$ cellules/ml) en IMDM + 10 %SVF. Incubation 20 min à 37°C puis centrifugation et évaluation de l'adhérence des cellules (contre une gamme témoin).
2) Révélation de l'anti-D fixé sur les plaques de Capture R par technique de type ELISA à l'aide d'anti IgG humaines-Péroxydase au 1/5000 (Sanofi Diagnostics Pasteur) après avoir lysé les cellules Jurkat CD16 au Tris -HCl 0,2M, Urée 6M, pH 5,3-5,5. Révélation OPD puis lecture de la densité optique (D.O.)à 492 nm.

Expression des résultats : on affecte une valeur arbitraire de 0 à 3 en fonction de la fixation et de l'étalement des cellules Jurkat CD16. Ces valeurs sont affectées à chaque intervalle de DO défini (de 0,1 en 0,1). On trace :

* soit une courbe : adhérence des cellules Jurkat (Y) en fonction de la quantité d'anti-D fixée sur les membranes d'hématies (X).
* soit un histogramme des «indices de fixation» correspondant pour chaque anticorps, à la somme de chaque valeur de fixation des cellules Jurkat (0 à 3) affectée par intervalle de DO (sur une portion commune à l'ensemble des anticorps testés).

Un exemple d'histogramme est présenté à la figure 2.
Les anticorps anti-Rh(D) d'isotype IgG1 (F60 et T125 YB2/0) montrent un indice de fixation proche de celui des IgG1 polyclonales (WinRho), alors que les anticorps témoins négatifs DF5 et AD1 ne se fixent pas. De la même façon, l'anticorps d'isotype IgG3 (F41) présente un bon indice de fixation, légèrement inférieur à celui des IgG3 purifiées à partir du polyclonal Winrho et supérieur à celui de l'anticorps AD3 (autre IgG3 testée et inefficace en essai clinique, en mélange avec AD1 (Biotest/LFB, 1997, non publié).

**Exemple 2 :**

**PRODUCTION D'UN ANTICORPS (Ac) ANTI-D RECOMBINANT**

**1- Isolement et amplification des ADNc codant pour les chaînes lourde et légère de l'Ac**

***1.1- Extraction des ARN et synthèse d'ADNc***

Les ARN totaux ont été extraits d'un clone producteur d'Ac anti-D (IgG G1/Kappa) obtenu par transformation EBV : T125 A2 (5/1) A2 (voir paragraphe 2, exemple 1)
Les cDNA correspondants ont été synthétisés par transcription reverse des ARN totaux à l'aide d'amorces oligo dT.

***1.2- Amplification de la région variable de la chaîne lourde de T125-A2 : séquence VH/T125-A2***

La séquence VH/T125-A2 est obtenue par amplification des cDNA de T125-A2 à l'aide des amorces suivantes :

- amorce A2VH5 localisée en 5'de la région leader du gène VH de T125-A2, introduit une séquence leader consensus (en gras) déduite de séquences leader déjà publiées et associées à des gènes VH appartenant à la même famille VH3-30 que le gène VH de T125-A2 ; cette séquence comporte également un site de restriction Eco RI (en italique) et une séquence de Kozak (soulignée) :

  A2VH5 (SEQ ID N°1) :
  5'- CTCTCC*GAATTC*GCCGCCACC**ATGGAGTTTGGGCTGAGCTGGGT** -3'

- amorce antisens GSP2ANP située en 5' de la région constante (CH) de T125-A2 : GSP2ANP (SEQ ID N°2) : 5'-GGAAGTAGTCCTTGACCAGGCAG -3'.

***1.3- Amplification de la région constante de T125-A2 : séquence CH/T125-A2***

La séquence CH/T125-A2 est obtenue par amplification des cDNA de T125-A2 à l'aide des amorces suivantes :

- amorce G1 localisée en S' de la région CH de T125-A2 :

  G1 (SEQ ID N°3) : 5'- CCCTCCACCAAGGGCCCATCGGTC -3'
  La première base G de la séquence CH est ici remplacée par un C (souligné) afin de recréer après clonage un site Eco RI (voir paragraphe 2.1.1)

- amorce antisens H3'Xba située en 3' du CH de T125-A2, introduit un site Xba I (souligné) en 3' de la séquence amplifiée:

  H3'Xba (SEQ ID N°4) :
  5'- GAGAGGTCTAGACTATTTACCCGGAGACAGGGAGAG -3'

*1.4- Amplification de la chaîne légère Kappa : séquence K/T125-A2*

La chaîne Kappa entière de T125-A2 (séquence K/T125-A2) est amplifiée à partir des cDNA de T125-A2 en utilisant les amorces suivantes :

- amorce A2VK3 située en 5' de la région leader du gène VK de T125-A2, introduit une séquence consensus (en gras) déduite de la séquence de plusieurs régions leader de gènes VK VH appartenant au même sous-groupe VK1 que le gène VK de T125-A2 ; cette séquence comporte également un site de restriction Eco RI (en italique) et une séquence de Kozak (soulignée) :

  A2VK3 (SEQ ID N°5) :

  5'- CCTACCG*AATTC*GCCGCCACC**ATGGACATGAGGGTCCCCGCTCA** -3'

- amorce antisens KSE1 localisée en 3' de Kappa, introduit un site Eco RI (souligné) :

  KSE1 (SEQ ID N°6) :

  5'- GGTGGTGAATTCCTAACACTCTCCCCTGTTGAAGCTCTT -3'.

La Fig. 1 schématise les stratégies d'amplification des chaînes lourde et légère de T125-A2.

2- Construction des vecteurs d'expression

*2.1- Vecteur d'expression chaîne lourde de T125-A2 : T125-H26*

La construction de T125-H26 est résumée Fig. 2. Elle s'effectue en deux temps : tout d'abord construction du vecteur intermédiaire V51-CH/T125-A2 par insertion de la région constante de T125-A2 dans le vecteur d'expression V51 dérivé de pCI-néo (Fig. 3) puis clonage de la région variable dans V51-CH/T125-A2.

*2.1.1 clonage de la région constante de T125-A2*

La séquence CH/T125-A2 amplifiée est insérée après phosphorylation au niveau du site Eco RI du vecteur V51 (Fig. 3). La ligation s'effectue après traitement préalable à la polymérase Klenow des extrémités cohésives Eco RI de V51 afin de les rendre « bout franc ».

L'amorce G1 utilisée pour l'amplification de CH/T125-A2 permet de recréer après son insertion dans V51 un site Eco RI en 5' de CH/T125-A2.

*2.1.2 clonage de région variable de T125-A2*

La séquence VH/T125-A2 obtenue par amplification est digérée par Eco RI et Apa I puis insérée au niveau des sites Eco RI et Apa I du vecteur V51-G1/T125-A2.

*2.2- Vecteur chaîne légère de T125-A2 : T125-K47*

La construction de T125-K47 est présentée Fig.4. La séquence K/T125-A2 obtenue par PCR est digérée par Eco RI et insérée au niveau du site Eco RI du vecteur d'expression V47 dérivé de pCI-néo (Fig. 5).

*2.3- Vecteur chaînes lourde et légère de T125-A2 : T125-IG24*

La construction de T125-IG24 est schématisée Fig.6. Ce vecteur qui contient les deux unités de transcription des chaînes lourde et kappa de T125-A2 est obtenu par insertion du fragment Sal I - Xho I de T125-K47 contenant l'unité de transcription de K/T125-A2 au niveau des sites Xho I et Sal I de T125-H26.

Ainsi les chaines lourdes et légères de T125-A2 sont exprimées sous la dépendance du promoteur CMV ; d'autres promoteurs peuvent être utilisés : RSV, promoteur chaine lourde IgG, LTR MMLV, HIV, β actine, etc...

***2.4- Vecteur leader spécifique chaînes lourde et légère de T125-A2 : T125-LS4***

Un second vecteur d'expression de T125-A2 est également construit dans lequel la séquence leader consensus de la chaîne Kappa est remplacée par la séquence réelle de la région leader de T125-A2 préalablement déterminée par séquençage de produits de « PCR 5'-RACE » (Rapid Amplification of cDNA 5' Ends).

La construction de ce vecteur T125-LS4 est décrite Fig. 7. Elle s'effectue en deux étapes : tout d'abord la construction d'un nouveau vecteur d'expression de la chaîne Kappa de T125-A2, T125-KLS18, puis l'assemblage du vecteur d'expression final, T125-LS4, contenant les deux unités de transcription chaîne légère modifiée et chaîne lourde.

*2.4.1 construction du vecteur T125-KLS18*

La partie 5' de la séquence leader consensus Kappa du vecteur T125-K47 est remplacée par la séquence leader spécifique de T125 (KLS/T125-A2) au cours d'une étape d'amplification de la séquence K/T125-A2 réalisée à l'aide des amorces suivantes :

- amorce A2VK9 , modifie la partie 5' de la région leader (en gras) et introduit un site Eco RI (souligné) ainsi qu'une séquence de Kozak (en italique) :

A2VK9 : 5'- CCTACC<u>GAATTC</u>*GCCGCCACC***ATGAGGGTCCCCGCTCAGCTC** - 3'

- amorce KSE1 (décrite au paragraphe 1.4)

Le vecteur T125-KLS18 est ensuite obtenu en remplaçant le fragment Eco RI de T125-K47 contenant la séquence K/T125-A2 d'origine par la nouvelle séquence KLS/T125-A2 digérée par Eco RI.

*2.4.2 construction du vecteur final T125-LS4*

Le fragment Sal I - Xho I de T125-KLS18 contenant la séquence modifiée KLS/T125-A2 est inséré dans T125-H26 au niveau des sites Xho I et Sal I.

**3- Production d'Ac anti-D dans la lignée YB2/0**

***3.1- Sans amplification génique***

Les deux vecteurs d'expression T125-IG24 et T125-LS4 ont été utilisés pour la transfection de cellules de la lignée YB2/0 (myélome de rat, lignée ATCC n° 1662).
Après transfection par électroporation et sélection des transformants en présence de G418 (sélection néo) plusieurs clones ont été isolés. La production en Ac anti-D recombinants est d'environ 0.2 $\mu$g/$10^6$ cellules/24h (*valeur obtenue pour le clone 3B2 de R270*). L'activité ADCC de cet Ac recombinant est supérieure ou égale à celle des témoins poly-D (figure 1). Les Ac produits à l'aide des deux vecteurs d'expression ne sont pas significativement différents en terme de niveau de production ou d'activité ADCC.

***3.2- Avec amplification génique***

Le système d'amplification génique mis en oeuvre repose sur la sélection de transformants résistant au méthotrexate (MTX). Il nécessite l'introduction préalable d'une unité de transcription codant pour l'enzyme DHFR (dihydrofolate réductase) dans le vecteur d'expression de l'Ac recombinant (SHITARI et al, 1994)

*3.2.1 construction du vecteur d'expression T125-dhfr 13*

Le schéma présenté Fig. 8 décrit la construction du vecteur d'expression de T125-A2 contenant le gène dhfr murin.

Un premier vecteur (V64) a été construit à partir d'un vecteur dérivé de pCI-néo, V43 (Fig. 9), en remplaçant, en 3' du promoteur SV40 et en 5' d'une séquence de polyadénylation synthétique, le gène néo (fragment Hind III- Csp 45 I) par le cDNA du gène dhfr murin (obtenu par amplification à partir du plasmide pMT2). Ce vecteur est ensuite

modifié de façon à créer un site Cla I en 5' de l'unité de transcription dhfr. Le fragment Cla I contenant l'unité de transcription dhfr est ensuite inséré au niveau du site Cla I de T125-LS4.

*3.2. 2 sélection en présence de MTX*

♦ 1ère stratégie :

Les cellules YB2/0 transfectées par électroporation avec le vecteur T125-dhfr13 sont sélectionnées en présence de G418. Les transformants producteurs d'Ac recombinant sont ensuite soumis à une sélection en présence de doses croissantes de MTX (de 25nM à 25μM). L'évolution de la production d'Ac recombinants, témoin du processus d'amplification génique, est suivie au cours des étapes de sélection en MTX. Les transformants résistants au MTX sont ensuite clonés par dilution limite. Le niveau et la stabilité de la production d'Ac recombinants est évaluée pour chaque clone obtenu. La productivité en anticorps anti-D après amplification génique est d'environ 13 (+/- 7) μg/ $10^6$cellules/24 h.

♦ 2ème stratégie:

Les cellules YB2/0 transfectées par électroporation avec vecteur T125-dhfr13 sont sélectionnées en présence de G418. Les meilleurs transformants producteurs d'Ac recombinant sont clonés par dilution limite avant sélection en présence de doses croissantes de MTX. L'évolution de la production de chaque clone, témoin du processus d'amplification génique, est suivie au cours des étapes de sélection en MTX. Le niveau et la stabilité de la production d'Ac recombinants est évaluée pour chaque clone MTX-résistant obtenu.

***4- Evaluation de l'activité de l'anticorps T125 exprimé dans YB2/0***

Après purification par chromatographie d'affinité sur protéine A Sépharose (Pharmacia) et dialyse en tampon Tris 25mM, NaCl 150mM, pH 7,4 , la concentration de l'anticorps T125 est déterminée par technique ELISA. L'activité biologique in vitro est ensuite mesurée par le test ADCC précédemment décrit. Les résultats sont présentés à la figure 1.

**EXEMPLE 3 : MISE EN EVIDENCE DE LA RELATION ENTRE STRUCTURE GLYCANNIQUE ET ACTIVITE DEPENDANTE DU FCγRIII :**

***1- Culture cellulaire en présence de Deoxymannojirimycine (DMM).***

Plusieurs travaux décrivent l'effet d'inhibiteurs enzymatiques sur la glycosylation des immunoglobulines et sur leur activité biologique. Une augmentation de l'activité ADCC est rapportée par ROTHMAN et al. 1989, augmentation non attribuable à une amélioration de l'affinité de l'anticorps pour sa cible. La modification de glycosylation provoquée par l'addition de DMM consiste en une inhibition de l'α1,2 mannosidase I présente dans le Golgi. Elle conduit à la production d'une proportion plus importante de structures polymannosylées, non fucosylées.
Différentes lignées productrices d'anticorps anti-Rh(D) ont été mises en présence de DMM et l'activité fonctionelle des anticorps monoclonaux produits a été évaluée sous forme de surnageants de culture ou après purification.
Les cellules (hétérohybrides ou lymphoblastoïdes) sont ensemencées entre 1 et $3x10^5$ cellules/ml, et cultivées en milieu de culture IMDM (Life Technologies) avec 10% de SVF et en présence de 20μg/ml de DMM (Sigma , Boehringer). Après 3 renouvellements de milieu, les surnageants de culture sont testés par ELISA IgG humaines puis par ADCC.

Tableau 2 : Effet de la culture en présence de DMM sur l'activité ADCC de différents anti-Rh(D)

| Echantillons | Activité ADCC En % de l'activité de poly D LFB51 | | Dose minimale de DMM nécessaire μg/ml |
|---|---|---|---|
| | Culture sans DMM | Culture en présence de DMM | |
| F60 | 109 | 113 | NT |
| D31 | 19 | 87 | 10 |
| DF5 | 26 | 62 | 20 |
| T125 RI(3) | 3 | 72 | 20 |
| T125-CHO | 0 | 105 | 5 |

(suite)

| Echantillons | Activité ADCC En % de l'activité de poly D LFB51 | | Dose minimale de DMM nécessaire µg/ml |
|---|---|---|---|
| | Culture sans DMM | Culture en présence de DMM | |
| NT= non testé | | | |

• La culture en présence de Deoxymannojirimycine (DMM) apporte une amélioration significative des résultats ADCC pour les anticorps faiblement actifs auparavant produits par :

| | |
|---|---|
| Un hybridome homme-souris | D31 |
| Une lignée lymphoblastoïde humaine | DF5 |
| Une lignée murine transfectée | T125 dans CHO |

• L'addition de DMM peut permettre de restaurer l'activité ADCC d'un anticorps issu du cloïde T 125 = T125 RI(3) (décrit en exemple 1) et qui a perdu cette activité par une culture prolongée.
• L'activité forte de l'anticorps produit par l'hétérohybridome F60 (dont l'obtention est décrite en exemple 1) n'est pas modifiée par culture en présence de DMM.

***2- Production d'anticorps anti-D recombinants par différentes lignées cellulaires :***

2.1- Préparation d'un vecteur d'expression pour l'anticorps DF5 :

La séquence nucléotidique de l'anticorps DF5, témoin négatif dans le test ADCC, est utilisée pour étudier la transfection de cet anticorps dans quelques lignées, parallèlement à la transfection de l'anticorps T125.
Les séquences codant pour l'Ac DF5 sont isolées et amplifiées selon les mêmes techniques mises en oeuvre pour l'Ac recombinant T125-A2.

- Les ADNc correspondants sont tout d'abord synthétisés à partir d'ARN totaux extraits du clone producteur d'Ac anti-D (IgG G1/Lambda) 2MDF5 obtenu par transformation EBV.
- L'amplification des chaînes lourde et légère est ensuite réalisée à partir de ces ADNc en utilisant les amorces présentées ci-dessous.
- Amplification de la région variable de la chaîne lourde de DF5 (séquence VH/DF5):
    - amorce DF5VH1 localisée en 5' de la région leader (en gras) du gène VH de DF5 (séquence publiée : Chouchane L et al.); cette amorce comporte également un site de restriction Eco RI (en italique) et une séquence de Kozak (soulignée) : DF5VH1 (SEQ ID N°8) :

      5'CTCTCC*GAATTC*<u>GCCGCCACC</u>**ATGGACTGGACCTGGAGGATCCTCTTTTTGGTGG**-3'

    - amorce antisens GSP2ANP située en 5' de la région constante (CH) déjà décrite au paragraphe 1.2 (exemple 2)
- Amplification de la région constante CH de DF5 (séquence CH/DF5) : amorces G1 et H3'Xba déjà décrites au paragraphe1.3 (exemple 2).
- Amplification de la chaîne légère Lambda de DF5 (séquence LBD/DF5) :
    - amorce DF5VLBD1 située en 5' région leader du gène VL de DF5, introduit une séquence consensus (en gras) déduite de la séquence de plusieurs régions leader de gènes VL appartenant au même sous-groupe VL1 que le gène VL de 2MDF5 ; cette séquence comporte également un site de restriction Eco RI (en italique) et une séquence de Kozak (soulignée) :

      DF5VLBD1 (SEQ ID N°9) :

5'CCTACCG*AATTC*GCCGCCACC**ATGGCCTGGTCTCCTCTCCTCCTCAC**-3'

- amorce antisens LSE1 localisée en 3' de Lambda, introduit un site Eco RI (souligné) :

LSE1 (SEQ ID N°10) :

5'- GAGGAGGAATTCACTATGAACATTCTGTAGGGGCCACTGTCTT -3'.

- La construction des vecteurs d'expression chaîne lourde (DF5-H31), chaîne légère (DF5-L10) et chaînes lourde et légère (DF5-IG1) de l'Ac DF5 est effectuée selon un schéma de construction similaires aux vecteurs exprimant l'Ac T125-A2. Toutes les séquences Leader d'origine (introduites au niveau des amorces d'amplification) sont conservées dans ces différents vecteurs.

2.2- Transfection de différentes lignées cellulaires avec les anticorps T125 et DF5

Les trois vecteurs d'expression T125-IG24, T125-LS4 et DF5-IgG1 sont utilisés pour la transfection de cellules de différentes lignées :
Des transfections stables ou transitoires sont réalisées par électroporation ou à l'aide de réactif de transfection.

Tableau 3 : Lignées cellulaires utilisées pour la transfection d'anticorps anti-Rh(D)

| Nom | Référence | Type cellulaire |
|---|---|---|
| CHO-K1 | ATCC CCL 61 | Ovaire de hamster chinois (epithelium like) |
| CHO-Lec10 | Fenouillet et al., 1996, Virology, 218, 224 - 231 | Ovaire de hamster chinois (epithelium like) |
| Jurkat | ATCC TIB-152 | Lymphocyte T humain (Leucémie T) |
| Molt-4 | ATCC CRL 1582 | Lymphocyte T humain (Leucémie lymphoblastique aiguë) |
| WIL2-NS | ATCC CRL 8155 | Lymphocyte B humain transformé EBV |
| Vero | ATCC CCL 81 | Rein de singe vert africain (fibroblaste like) |
| COS-7 | ATCC CRL 1651 | Rein de singe vert africain transformé SV40 (fibroblaste like) |
| 293-HEK | ATCC CRL 1573 | Rein embryonnaire humain primaire transformé par ADN adénovirus 5 défectif |
| YB2/0 | ATCC CRL 1662 | Myélome de rat non sécréteur |
| BHK-21 | ATCC CCL 10 | Rein de hamster nouveau-né (fibroblaste like) |
| K6H6-B5 | ATCC CRL 1823 | hétéromyélome homme-souris non sécréteur |
| NSO | ECACC 85110503 | myélome souris non sécréteur (lymphoblate like) |
| SP2/0- Ag 14 | ECACC 85072401 | Hybridome souris x souris non sécréteur |
| CHO Lec-1 | ATCC CRL 1735 | Ovaire de hamster chinois |
| CHO dhfr- | ECACC 94060607 | Ovaire de hamster chinois |
| CHO Pro-5 | ATCC CRL 1781 | Ovaire de hamster chinois |
| P3X63 Ag8.653 | ATCC CRL 1580 | Myelome de souris non sécréteur |

Après sélection des transformants en présence de G418 (sélection néo) plusieurs clones ont été isolés.
La modification d'activité effectrice d'un anticorps monoclonal humanisé en fonction de la cellule d'expression a été décrite par CROWE et al (1992), avec les lignées cellulaires CHO, NSO, YB2/O.
Les résultats obtenus ici confirment l'importance de la lignée cellulaire d'expression vis à vis des caractéristiques fonctionnelles de l'anticorps à produire. Parmi les cellules testées, seules les lignées Vero, YB2/0 et CHO Lec-1 permettent d'exprimer des anticorps monoclonaux anti-Rh(D) recombinants avec une activité lytique forte dans le test ADCC

(voir exemple 1 et tableau 3).

Tableau 3 :Activité ADCC des anticorps DF5 et T125 obtenus par transfection dans différentes lignées cellulaires. Les résultats sont exprimés en pourcentage de l'activité de l'anticorps polyclonal de référence : Poly-D LFB 51

| | | Lignées cellulaires transfectées | | | | | | | | |
|---|---|---|---|---|---|---|---|---|---|---|
| | | CHO-K1 | CHO-Lec10 | Wil-2 | Jurkat | Vero | Molt-4 | COS-7 | 293-HEK | YB2/0 |
| anticorps | T125 | 7 +/-8 n=13 | 22 +/-6 n=11 | 3 +/-5 n=12 | 6 +/-8 n=7 | 90 +/-21 n=5 | 0 n=1 | 13 +/-2 n=4 | 16 +/-13 n=12 | 114 +/-28 n=54 |
| | DF5 | NT | 51 +/-19 n=3 | NT | NT | 72 +/-17 n=5 | NT | 21 +/-4 n=4 | 12 +/-14 n=12 | 94 +/-15 n=15 |

| | | Lignées cellulaires transfectées | | | | | | | |
|---|---|---|---|---|---|---|---|---|---|
| | | NS0 | BHK | CHO-Lec 1 | SP2/O-Ag14 | K6H6-B5 | CHO Pro-5 | CHO dhfr- | P3X63Ag8.653 |
| anticorps | T125 | 6 +/- 8 n= 3 | 13 +/- 5 n= 4 | 106 +/- 60 n= 4 | 0 +/- 0 n= 6 | 9 +/- 8 n= 3 | 3 +/- 3 n= 4 | 13 +/- 8 n= 12 | 34 +/- 8 n= 9 |

***3*- étude des structures glycanniques**

La caractérisation des structures glycanniques de l'anticorps anti Rh-D a été réalisée sur quatre produits purifiés ayant une activité ADCC (F60, et trois protéines recombinantes issues de T125) en comparaison avec deux produits purifiés inactifs ou très faiblement actifs dans le test ADCC selon l'invention (D31 et DF5).
En pratique, les oligosaccharides sont séparés de la protéine par une déglycosylation enzymatique spécifique par la PNGase F au niveau de l'Asn 297. Les oligosaccharides ainsi libérés sont marqués par un fluorophore, séparés et identifiés par différentes techniques complémentaires qui permettent :

- Une caractérisation fine des structures glycaniques par spectrométrie de masse à desorption laser assistée par matrice (MALDI) par comparaison des masses expérimentales avec les masses théoriques.
- La détermination du taux de sialylation par HPLC d'échange d'ions (GlycoSep **C**)
- La séparation et la quantification des formes oligosaccharidiques suivant des critères d'hydrophilicité par HPLC en phase normal (GlycoSep N)
- La séparation et la quantification des oligosaccharides par électrophorèse capillaire à détection de fluorescence induite par laser (HPCE-LIF).

**1) Caractérisation des glycanes des formes actives**

Les différentes formes actives étudiées sont F60 et trois anticorps recombinants, R 290, R 297 et R 270, issus de T125 et produits dans YB2/0. La caractérisation fine des structures glycaniques par spectrométrie de masse (figure 7) montre que ces formes sont toutes de type biantennées. Dans le cas de R 270 la forme majoritaire est de type agalactosylée non fucosylée (G0, masse exp. 1459.37 Da, fig 1). Trois autres structures sont identifiées : agalactosylée fucosylée (G0F à 1605.41 Da), monogalactosylée non fucosylée (G1 à 1621.26 Da) et monogalactosylée fucosylée (G1F à 1767.43 Da) minoritaire. Ces quatre même structures sont caractéristiques de R 290, F 60 et R 297 (Figure 1). Ces quatre anticorps actifs en ADCC sont également caractérisés par l'absence d'oligosaccharides possédant un résidu N-acétylglucosamine en bissectrice.
La quantification des structures glycaniques par les différentes techniques d'HPLC et HPCE-LIF (Tableau I) confirme la présence des quatre formes identifiées en masse : G0, G0F, G1 et G1F. Le taux de sialylation est très faible, notamment pour les produits recombinants, de 1 à 9.4 % ce qui est confirmé par la similitude des spectres de masse obtenus avant et après désialylation enzymatique. Le taux de fucosylation varie de 34 à 59 %.

**2) Formes inactives**

Les différentes formes inactives étudiées sont D31 et DF5. La quantification des structures glycaniques par les différentes techniques chromatographiques et d'électrophorèse capillaire (Tableau I) révèle, pour ces deux anticorps, un taux de sialylation proche de 50%, et un taux de fucosylation de 88 et 100 % pour D31 et DF5, respectivement. Ces taux de sialylation et fucosylation sont très supérieurs à ceux obtenus à partir des formes actives.
La caractérisation des structures glycaniques montre que la forme majoritaire est, pour les deux anticorps, de type bianténnée monosialylée bigalactosylée fucosylée (G2S1F, tableau I). La caractérisation par spectrométrie de masse de D31 (figure 7) révèle que les formes neutres sont majoritairement de type monogalactosylée fucosylée (G1F à 1767.43 Da) et bigalactosylée fucosylée (G2F à 1929.66 Da).
L'anticorps inactif DF5 est caractérisé par la présence d'oligosaccharides possédant un résidu GlcNAc intercalaire. En particulier, l'analyse en masse (figure 8) révèle la présence d'une forme neutre majoritaire de type monogalactosylée fucosylée Bisec-GlcNAc intercalaire (G1FB à 1851.03 Da). Par contre ces formes structurales sont non détectables ou présentes à l'état de trace sur les anticorps actifs étudiés.
L'activité ADCC du D31 après action du DMM passe de 10% à 60%. Les structures glycaniques du D31 DMM diffèrent de celles de D31 par la présence de formes oligomannoses (Man 5, Man 6 et Man 7) (voir figure 9).

**3) Conclusion**

Les différents anticorps actifs sont modifiés sur l'Asn 297 par des N-glycosylations de type bianténné et/ou oligomanosidiques. Pour les formes bianténnées il s'agit de structures courtes très faiblement sialylées, faiblement fucosylées, faiblement galactosylées et sans GlcNAc intercalaire.

**REFERENCES**

Boylston, J.M., Gardner, B., Anderson, R.L., and Hughes-Jones, N.C. Production of human IgM anti-D in tissue culture by EB virus-transformed lymphocytes. Scand. J. Immunol. 12 : 355-358 (1980).

Bron, D., Feinberg, M.B., Teng, N.N.H. and Kaplan, H.S. Production of Human Monoclonal IgG Antibodies against Rhesus (D) Antigen. Proc. Nat. Acad. Sci. USA 81 : 3214-3217 (1984).

Chouchane, L., Van Spronsen, A., Breyer, J., Guglielmi, P., and Strosberg, AD. Molecular characterization of a human anti-Rh(D) antibody with a DH segment encoded by a germ-line sequence. Eur. J. Biochem. 1 ;207(3) : 1115-1121 (1992).

Crawford, D.H., Barlow, M.J., Harrison, J.F., Winger, L. and Huehns, E.R. Production of human monoclonal antibody to rhesus D antigen. Lancet, i : 386-388 (1983).

Doyle, A., Jones, T.J., Bidwell, J.L, and Bradley, B.A. In vitro development of human monoclonal antibody secreting plasmacytomas. Hum. Immunol. 13 : 199-209 (1985).

Edelman, L., Margaritte, C., Chaabihi, H., Monchâtre, E., Blanchard, D., Cardona, A., Morin, F., Dumas, G., Petres, S. and Kaczorek, M. Obtaining a functional recombinant anti-rhesus (D) antibody using the baculovirus-insect cell expression System. Immunology, Vol. 91 (1), 13-19 (1997).

Foung, S.K.H., Blunt, J.A., Wu, P.S., Ahearn, P., Winn, L.C., Engleman, E.G. and Grumet, F.C. Human Monoclonal Antibodies to Rho (D). Vox Sang. 53 : 44-47 (1987).

Goossens, D., Champomier, F., Rouger, P., and Salmon, C. Human Monoclonal Antibodies against Blood Group Antigens : Preparation of a series of stable EBV immortalized B clones producing high levels of antibody of different isotypes and specificities. J. Immunol. Methods 101 : 193-200 (1987).

Issitt, P.D. Genetics of the Rh Blood Group System : Some Current Concepts. Med. Lab. Sci. 45 : 395-404 (1988).

Jefferis, R, Lund, J., Mizutani, H., Nakagawa, H., Kawazoe, Y., Arata, Y. and Takahashi, N. A comparative study of the N-linked oligosaccharides structure of human IgG Subclass proteins. Biochem. J., 268 : 529-537 (1990).

Koskimies, S. Human Lymphoblastoid Cell Line Producing Specific Antibody against Rh-Antigen D. Scand. Immunol. 11 : 73-77 (1980).

Kumpel, B.M., Goodrick, M.J., Pamphilon, D.H., Fraser, I.D., Poole G.D., Morse, C., Standen, G.R., Chapman, G.E., Thomas, D.P. and Anstee, D.J. Human Rh D monoclonal antibodies (BRAD-3 and BRAD-5) Cause Accelerated Clearance of Rh D + Red blood Cells and Suppression of Rh D Immunization in Rh D Volunteers. Blood, Vol. 86, n° 5, 1701-1709 (1995).

Kumpel, B.M., Poole, G.D. and Bradley, B.A. Human Monoclonal Anti-D Antibodies. I. Their Production, Serology, Quantitation and Potential Use as Blood Grouping Reagents. Brit. J. Haemat. 71 : 125-129 (1989a).

Kumpel, B.M., Rademacher, T.W., Rook, G.A.W., Williams, P.J., Wilson, I.B.M. Galacatosylation of human IgG anti-D produced by EBV-transformed B lymphoblastoid cell lines is dependent on culture method and affects Fc receptor mediated functional activity. Hum. Antibodies and Hybridomas, 5 : 143-151 (1994).

Leatherbarrow, R.J., Rademacher, T.W., Dwek, R.A., Woof, J.M., Clark, A., Burton, D.R., Richardson, N. and Feinstein, A. Effector functions of monoclonal aglycosylated mouse IgG2a ; binding and activation of complement component C1 and interaction with human Fc receptor. Molec. Immun. 22, 407-415 (1985).

Lomas, C., Tippett, P., Thompson, K.M., Melamed, M.D. and Hughes-Jones, N.C. Demonstration of seven epitopes on the Rh antigen D using human monoclonal anti-D antibodies and red cells from D categories. Vox Sang. 57 : 261-264 (1989).

Lund, J., Takahaski, N., Nakagawa, H., Goodall, M., Bentley, T., Hindley, S.A., Tyler, R. and Jefferis, R. Control of IgG/Fc glycosylation : a comparison of oligosaccharides from chimeric human/mouse and mouse subclass immunoglobulin G5. Molec. Immun. 30, n° 8, 741-748 (1993).

Lund, J., Tanaka, T., Takahashi, N., Sarmay, G., Arata, Y. and Jefferis, R. A protein structural change in aglycosylated IgG3 correlates with loss of hu Fc□RI and hu FcγRIII binding and/or activation. Molec. Immun. 27, 1145-1153 (1990).

Ma, J.K. and Hein, M.B. Immunotherapeutic potential of antibodies produced in plants. Trends Biotechnol. 13, 522-527 (1995).

Mc Cann-Carter, M.C., Bruce, M., Shaw, E.M., Thorpe, S.J., Sweeney, G.M., Armstrong, S.S. and James, K. The production and evaluation of two human monoclonal anti-D antibodies. Transf. Med. 3 : 187-194 (1993).

Melamed, M.D., Gordon, J., Ley, S.J., Edgar, D. and Hughes-Jones, N.C. Senescence of a human lymphoblastoid clone producing anti-Rhesus (D) Eur. J. Immunol. 115 : 742-746 (1985).

Parekh, R.B., Dwek, R.A., Sutton, B.J., Fernandes, D.L., Leung, A., Stanworth, D., Rademacher, T.W., Mizuochi, T., Taniguchi, T., Matsuta, K., Takeuchi, F., Nagano, Y., Miyamoto, T. and Kobata, A. Asssociation of rheumatoid arthritis and primary osteoarthritis with changes in the glycosylation pattern of total serum IgG. Nature, 316 : 452-457

(1985).

Rothman, RJ., Perussia, B., Herlyn, D. and Warren, L. Antibody-dependent cytotoxicity mediated by natural killer cells is enhanced by castanospermine-induced alterations of IgG glycosylation. Mol. Immunol. 26(12) : 1113-1123 (1989).

Shitara K., Nakamura K., Tokutake-Tanaka Y., Fukushima M., and Hanai N. A new vector for the high level expression of chimeric antibodies to myeloma cells. J. Immunol. Methods 167 : 271-278 (1994).

Thompson, K.M., Hough, D.W., Maddison, P.J., Melamed, M.D. and Hughes-Jones, N.C. Production of human monoclonal IgG and IgM antibodies with anti-D (rhesus) specificity using heterohybridomas. Immunology 58 : 157-160 (1986)

Thomson, A., Contreras, M., Gorick, B., Kumpel, B., Chapman, G.E. Lane, R.S., Teesdale, P. Hughes-Jones, N.C. and Mollison, P.L. Clearance of Rh D-positive red cells with monoclonal anti-D. Lancet 336 : 1147-1150 (1990).

Tippett, P. Sub-divisions of the Rh (D) antigen. Med. Lab. Sci. 45 : 88-93 (1988).

Ware, R.E. and Zimmerman, S.A. Anti-D : Mechanisms of action. Seminars in Hematology, vol. 35, n° 1, supp. 1 : 14-22 (1998).

Yu, I.P.C., Miller, W.J., Silberklang, M., Mark, G.E., Ellis, R.W., Huang, L., Glushka, J., Van Halbeek, H., Zhu, J. and Alhadeff, J.A. Structural characterization of the N-Glycans of a humanized anti-CD18 murine immunoglobulin G. Arch. Biochem. Biophys. 308, 387-399 (1994).

Zupanska, B., Thompson, E., Brojer, E. and Merry, A.H. Phagocytosis of Erythrocytes Sensitized with Known Amounts of IgG1 and IgG3 anti-Rh antibodies. Vox Sang. 53 : 96-101 (1987).

## Revendications

**1.** Procédé de préparation d'un anticorps monoclonal capable d'activer les cellules effectrices exprimant le FcγRIII **caractérisé en ce qu'**il comprend les étapes suivantes :

a) purification d'anticorps monoclonaux obtenus à partir de différents clones provenant de lignées cellulaires sélectionnées parmi les lignées cellulaires hétérohybrides obtenues par fusion de lymphocytes B humains provenant de sujets immunisés avec des cellules de myélome K6H6-B5 (ATCC n°CRL 1823) et la lignée cellulaire YB2/0 transfectée à l'aide d'un vecteur contenant le gène codant pour une immunoglobuline humaine de type IgG ;
b) addition de chaque anticorps obtenu à l'étape a) dans un mélange réactionnel distinct comprenant les cellules cibles desdits anticorps, des cellules effectrices comprenant des cellules exprimant le FcγRIII, des IgG polyvalentes humaines thérapeutiques,
c) détermination du pourcentage de lyse des cellules cibles et sélection des anticorps monoclonaux qui activent les cellules effectrices provoquant une lyse significative des cellules cibles (activité ADCC de type FcγRIII),

**caractérisé en ce que** les récepteurs FcγRI sont saturés par l'addition d'IgG polyvalentes humaines thérapeutiques.

**2.** Procédé selon la revendication précédente, **caractérisé en ce qu'**on sélectionne les anticorps présentant un taux ADCC de type FcγRIII supérieur à 60%, 70%, 80% ou de préférence supérieur à 90%.

**3.** Procédé selon l'une des revendications précédentes, **caractérisé en ce que** les cellules cibles sont des hématies traitées à la papaïne.

**4.** Procédé selon l'une des revendications précédentes, **caractérisé en ce qu'**on dépose par puits :

- 100µl d'anticorps monoclonaux purifiés à des concentrations de 20 à 200 ng/ml,
- 25µl d'hématies papaïnées, soit environ 1x$10^6$ cellules,
- 25 µl de cellules effectrices, soit environ 2x$10^6$ cellules,

- et 50 μl d'IgG polyvalentes, notamment de TEGELINE™, à une concentration comprise entre 1 et 20 mg/ml.

**5.** Procédé selon l'une des revendications précédentes, **caractérisé en ce que** l'on compare la quantité de lyse des cellules cibles à deux témoins positifs consistant en un composé chimique tel que du $NH_4Cl$ et un anticorps de référence actif in vivo et à un témoin négatif consistant en un anticorps inactif in vivo.

**6.** Procédé selon l'une des revendications précédentes, **caractérisé en ce que** les anticorps monoclonaux sont des anti-Rh(D) et lès cellules cibles sont des hématies rhésus D.

**7.** Procédé selon la revendication 6, **caractérisé en ce qu'**on, utilise les anticorps, polyclonaux d'origine commerciale comme témoins positifs et un anticorps monoclonal inapte à induire une clairance in vivo comme témoin négatif.

## Patentansprüche

**1.** Verfahren zur Herstellung eines monoklonalen Antikörpers, der in der Lage ist,
die das FcγRIII exprimierenden Effektorzellen zu aktivieren, **dadurch gekennzeichnet, dass** es die folgenden Schritte umfasst:

a) Reinigung von monoklonalen Antikörpern, die, ausgehend von verschiedenen Klonen, welche von Zelllinien, ausgewählt aus den Heterohybrid-Zelllinien, erhalten durch Fusion von humanen B-Lymphozyten, die von mit Myelom-Zellen K6H6-B5 (ATCC Nr. CRL 1823) immunisierten Probanden stammen, und der mit Hilfe eines Vektors, welcher das für ein humanes Immunglobulin vom Typ IgG kodierende Gen umfasst, transfizierten Zelllinie YB2/0 stammen, erhalten worden sind;
b) Hinzufügen von jedem in Schritt a) erhaltenen Antikörper zu einer jeweils separaten Reaktionsmischung, die Zielzellen der Antikörper, Effektorzellen, umfassend Zellen, welche das FcγRIII exprimieren, therapeutische humane polyvalente IgG umfasst,
c) Bestimmung des Prozentsatzes von Lyse der Zielzellen und Auswahl der monoklonalen Antikörper, die die Effektorzellen aktivieren, welche eine signifikante Lyse der Zielzellen hervorrufen (ADCC-Aktivität vom Typ FcγRIII),

**dadurch gekennzeichnet, dass** die Rezeptoren FcγRI durch Hinzufügen von therapeutischen humanen polyvalenten IgG gesättigt werden.

**2.** Verfahren nach vorangegangenem Anspruch, **dadurch gekennzeichnet, dass** man die Antikörper auswählt, die einen Grad von ADCC vom Typ FcγRIII über 60%, 70%, 80% oder vorzugsweise über 90% aufweisen.

**3.** Verfahren nach einem der vorangegangenen Ansprüche, **dadurch gekennzeichnet, dass** die Zielzellen mit Papain behandelte Erythrozyten sind.

**4.** Verfahren nach einem der vorangegangenen Ansprüche, **dadurch gekennzeichnet, dass** man pro Vertiefung vorlegt:

- 100 μl gereinigte monoklonale Antikörper in Konzentrationen von 20 bis 200 ng/ml,
- 25 μl mit Papain behandelte Erythrozyten, entsprechend etwa 1 x $10^6$ Zellen,
- 25 μl Effektorzellen, entsprechend etwa 2 x $10^6$ Zellen,
- und 50 μl polyvalente IgG, insbesondere TEGELINETM, in einer Konzentration zwischen 1 und 20 mg/ml.

**5.** Verfahren nach einem der vorangegangenen Ansprüche, **dadurch gekennzeichnet, dass** man das Ausmaß von Lyse der Zielzellen mit zwei positiven Vergleichsproben, bestehend aus einer chemischen Verbindung, wie $NH_4Cl$, und einem in vivo aktiven Referenzantikörper, und mit einer negativen Vergleichsprobe, bestehend aus einem in vivo inaktiven Antikörper, vergleicht.

**6.** Verfahren nach einem der vorangegangenen Antikörper, **dadurch gekennzeichnet, dass** die monoklonalen Antikörper anti-Rh(D) sind und die Zielzellen Rhesus D-Erythrozyten sind.

**7.** Verfahren nach Anspruch 6, **dadurch gekennzeichnet, dass** man polyklonale Antikörper kommerzieller Herkunft als positive Vergleichsproben und einen monoklonalen Antikörper, der nicht in der Lage ist, in vivo eine Clearance

zu induzieren, als negative Vergleichsprobe einsetzt.

**Claims**

1. Method for preparing a monoclonal antibody capable of activating effector cells expressing FcγRIII, **characterized in that** it comprises the following steps:

   a) purifying monoclonal antibodies obtained from various clones originating from cell lines selected from heterohybrid cell lines obtained by fusion of human B lymphocytes originating from immunized individuals with K6H6-B5 myeloma cells (ATCC No. CRL 1823) and the YB2/0 cell line transfected with a vector containing the gene encoding a human IgG immunoglobulin;
   b) adding each antibody obtained in step a) to a different reaction mixture comprising the target cells for said antibodies, effector cells comprising cells expressing FcγRIII, therapeutic human polyvalent IgGs,
   c) determining the percentage lysis of the target cells and selecting the monoclonal antibodies which activate the effector cells causing significant lysis of the target cells (FcγRIII-type ADCC activity),

   **characterized in that** the FcγRI receptors are saturated by the addition of therapeutic human polyvalent IgGs.

2. Method according to the preceding claim, **characterized in that** the antibodies which exhibit an FcγRIII-type ADCC level greater than 60%, 70%, 80%, or preferably greater than 90%, are selected.

3. Method according to one of the preceding claims, **characterized in that** the target cells are red blood cells treated with papain.

4. Method according to one of the preceding claims, **characterized in that** the following are deposited per well:

   - 100 μl of purified monoclonal antibodies at concentrations of 20 to 200 ng/ml,
   - 25 μl of papain-treated red blood cells, i.e. approximately 1 x $10^6$ cells,
   - 25 μl of effector cells, i.e. approximately 2 x $10^6$ cells,
   - and 50 μl of polyvalent IgGs, in particular of TEGELINE™, at a concentration of between 1 and 20 mg/ml.

5. Method according to one of the preceding claims, **characterized in that** the amount of target cell lysis is compared to two positive controls consisting of a chemical compound such as $NH_4C1$ and a reference antibody active *in vivo,* and to a negative control consisting of an antibody inactive in *vivo.*

6. Method according to one of the preceding claims, **characterized in that** the monoclonal antibodies are anti-Rh(D)s and the target cells are rhesus D red blood cells.

7. Method according to Claim 6, **characterized in that** polyclonal antibodies of commercial origin are used as positive controls and a monoclonal antibody incapable of inducing clearance in vivo is used as a negative control.

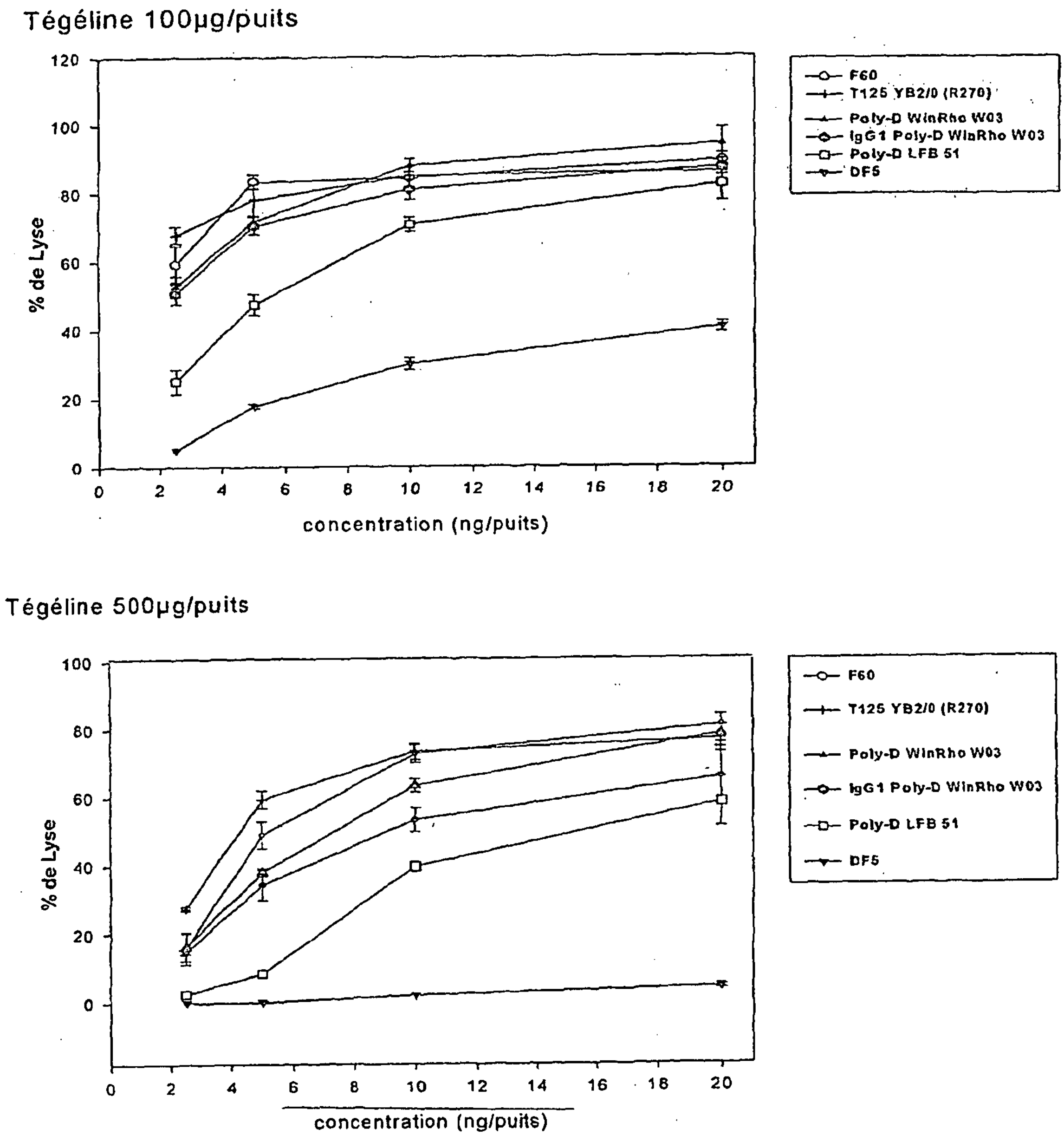
Tégéline 100µg/puits
% de Lyse
concentration (ng/puits)
F60
T125 YB2/0 (R270)
Poly-D WinRho W03
IgG1 Poly-D WinRho W03
Poly-D LFB 51
DF5
Tégéline 500µg/puits
% de Lyse
concentration (ng/puits)
F60
T125 YB2/0 (R270)
Poly-D WinRho W03
IgG1 Poly-D WinRho W03
Poly-D LFB 51
DF5

**FIGURE 1**

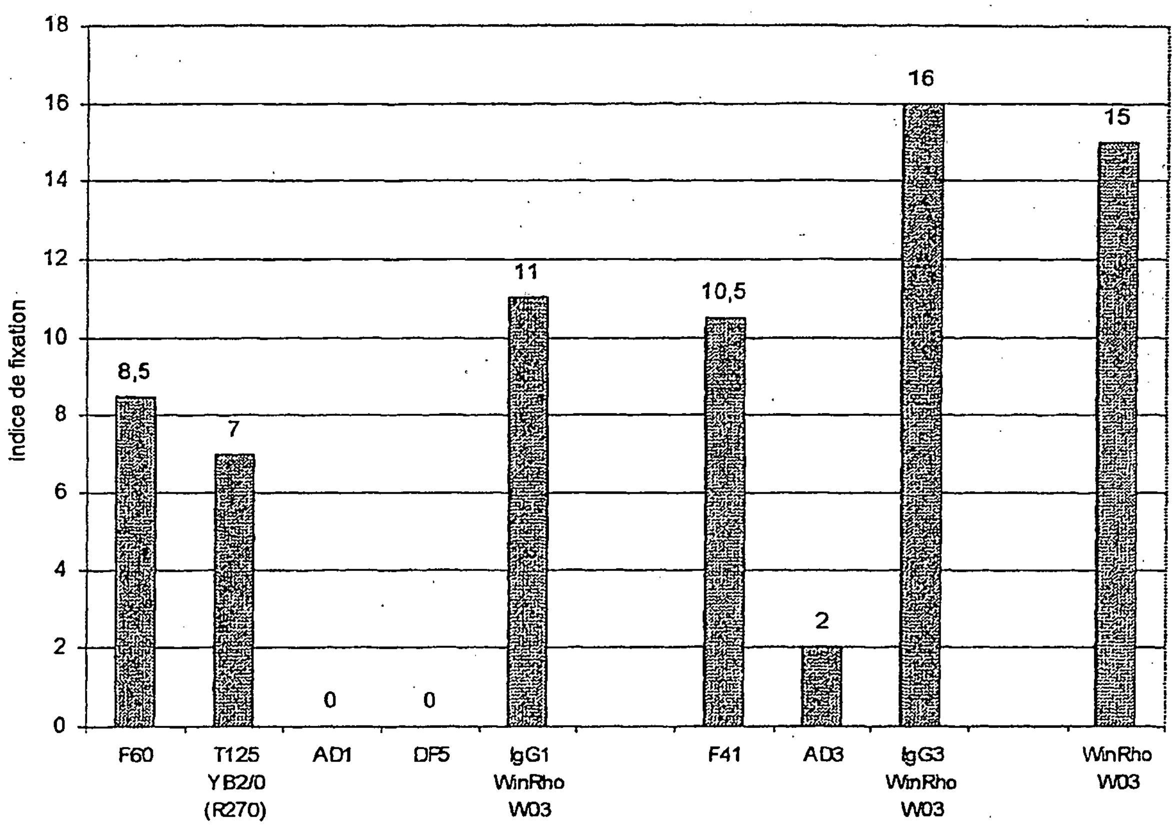

Indice de fixation
18
16
14
12
10
8
6
4
2
0
8,5
7
0
0
11
10,5
2
16
15
F60
T125 YB2/0 (R270)
AD1
DF5
IgG1 WinRho W03
F41
AD3
IgG3 WinRho W03
WinRho W03


FIGURE 2

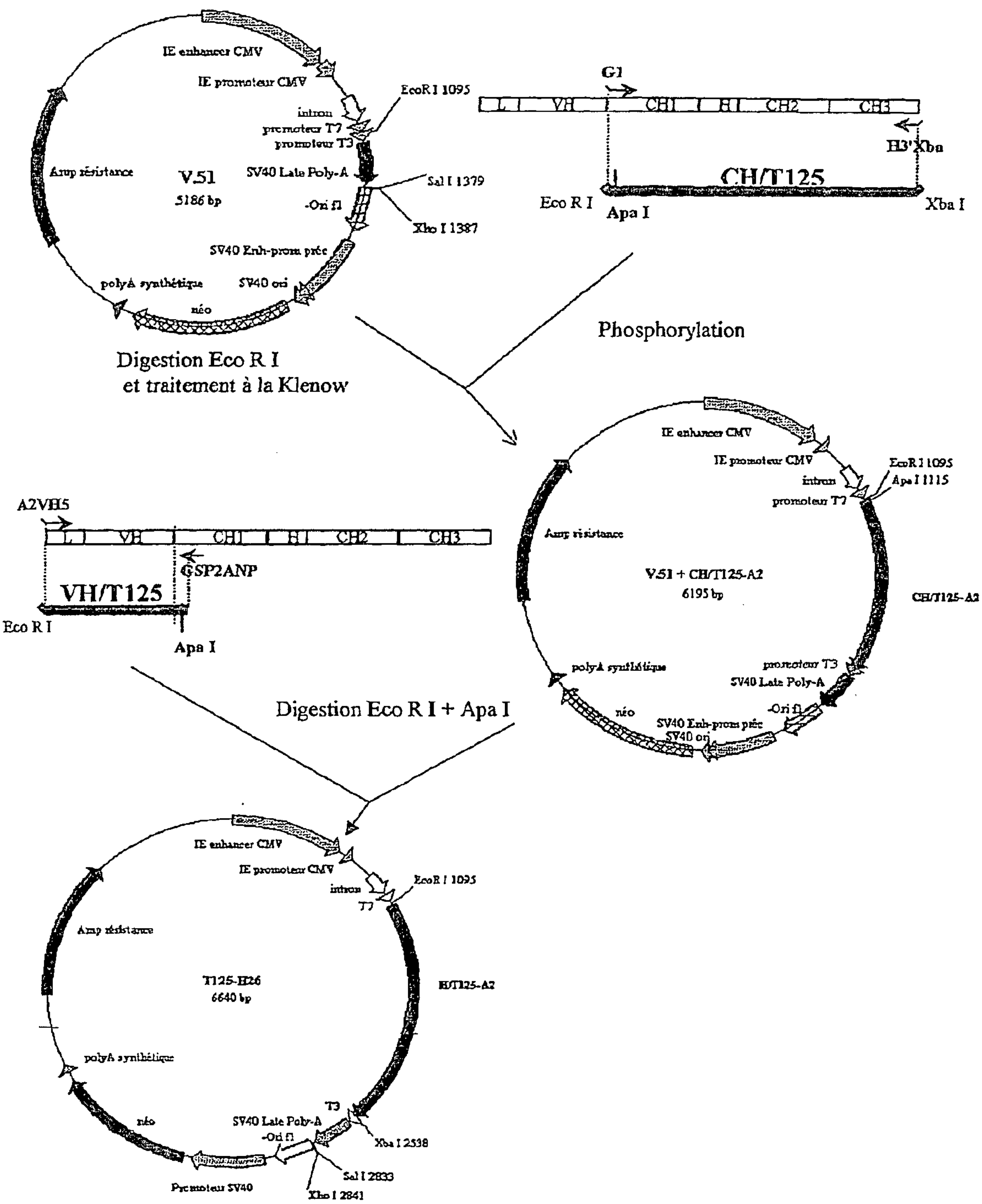
IE enhancer CMV
IE promoteur CMV
intron
promoteur T7
promoteur T3
EcoR I 1095
Amp résistance
V.51
5186 bp
SV40 Late Poly-A
Sal I 1379
-Ori f1
Xho I 1387
SV40 Enh-prom préc
SV40 ori
polyA synthétique
néo
G1
L
VH
CH1
H1
CH2
CH3
H3'Xba
CH/T125
Eco R I
Apa I
Xba I
Phosphorylation
Digestion Eco R I
et traitement à la Klenow
IE enhancer CMV
IE promoteur CMV
EcoR I 1095
intron
Apa I 1115
promoteur T7
Amp résistance
V.51 + CH/T125-A2
6195 bp
CH/T125-A2
promoteur T3
SV40 Late Poly-A
-Ori f1
polyA synthétique
néo
SV40 Enh-prom préc
SV40 ori
A2VH5
L
VH
CH1
H1
CH2
CH3
GSP2ANP
VH/T125
Eco R I
Apa I
Digestion Eco R I + Apa I
IE enhancer CMV
IE promoteur CMV
EcoR I 1095
intron
T7
Amp résistance
T125-H26
6640 bp
H/T125-A2
polyA synthétique
T3
néo
SV40 Late Poly-A
-Ori f1
Xba I 2538
Sal I 2833
Promoteur SV40
Xho I 2841

FIGURE 3

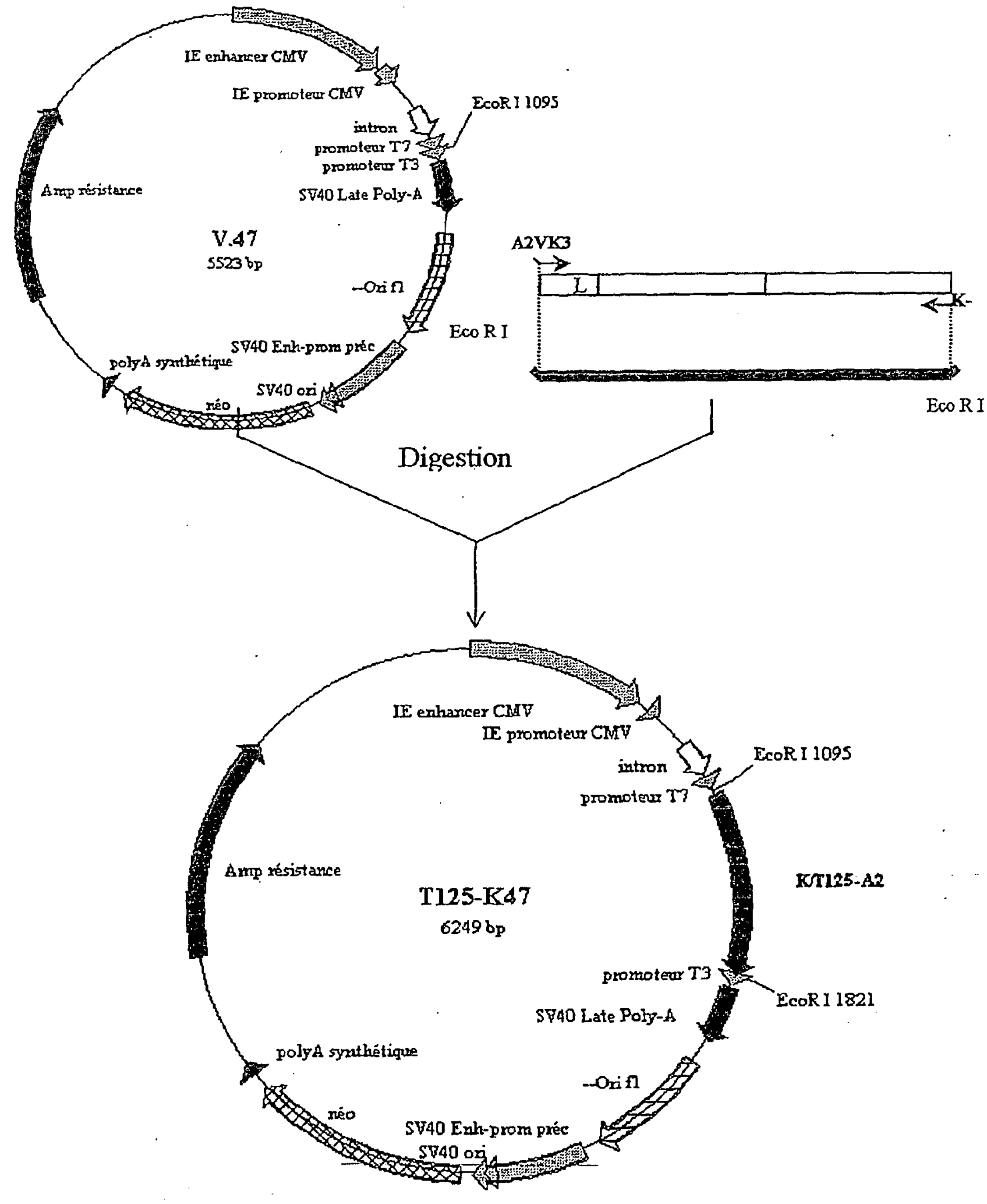

IE enhancer CMV
IE promoteur CMV
EcoR I 1095
intron
promoteur T7
promoteur T3
Amp résistance
SV40 Late Poly-A
V.47
5523 bp
--Ori f1
Eco R I
SV40 Enh-prom préc
polyA synthétique
SV40 ori
néo
A2VK3
L
K-
Eco R I
Digestion
IE enhancer CMV
IE promoteur CMV
EcoR I 1095
intron
promoteur T7
Amp résistance
T125-K47
6249 bp
K/T125-A2
promoteur T3
EcoR I 1821
SV40 Late Poly-A
polyA synthétique
--Ori f1
néo
SV40 Enh-prom préc
SV40 ori


FIGURE 4

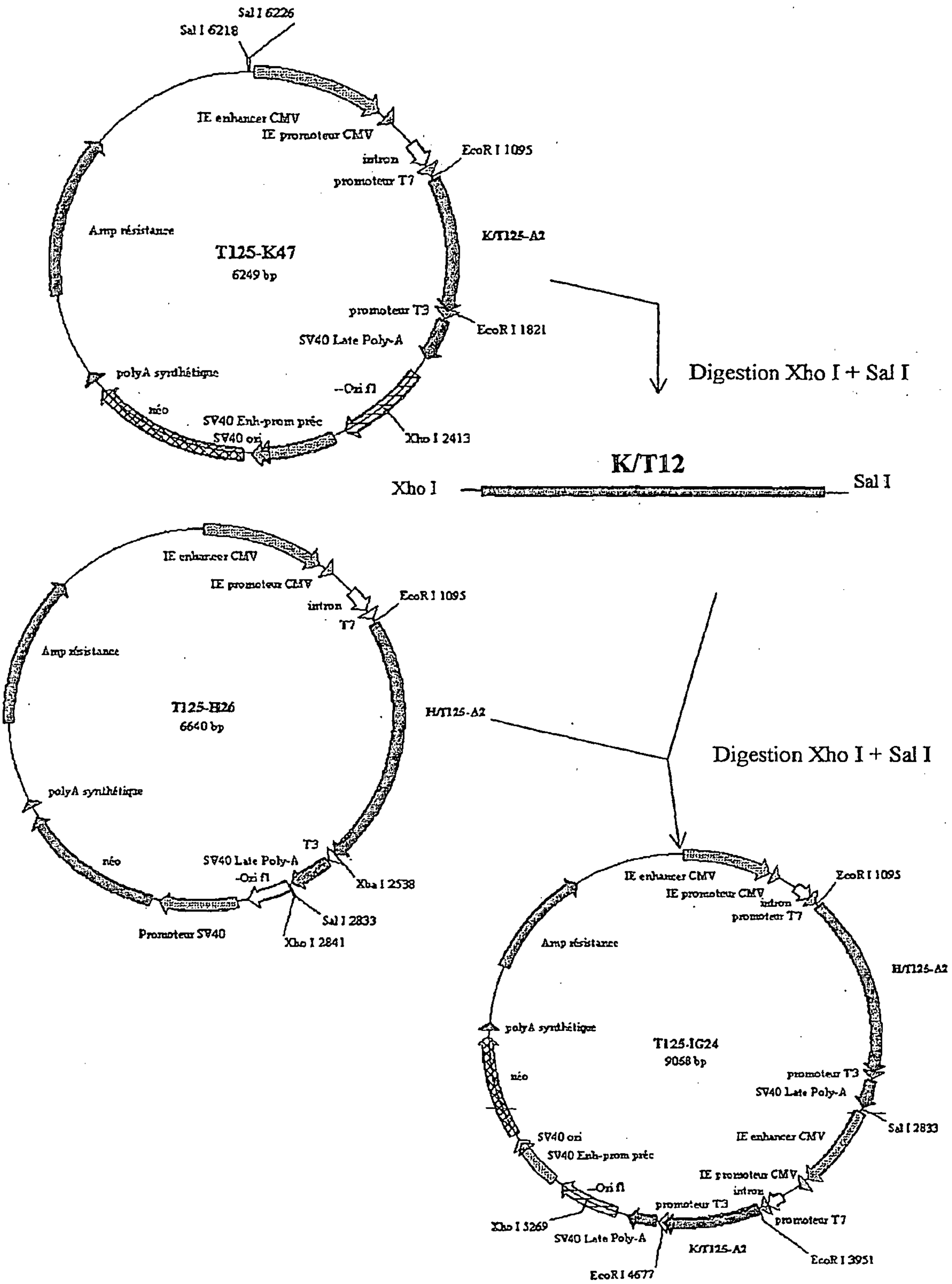
Sal I 6226
Sal I 6218
IE enhancer CMV
IE promoteur CMV
intron
EcoR I 1095
promoteur T7
Amp résistance
T125-K47
6249 bp
K/T125-A2
promoteur T3
EcoR I 1821
SV40 Late Poly-A
polyA synthétique
Ori f1
néo
SV40 Enh-prom préc
SV40 ori
Xho I 2413
Digestion Xho I + Sal I
K/T12
Xho I
Sal I
IE enhancer CMV
IE promoteur CMV
intron
EcoR I 1095
T7
Amp résistance
T125-H26
6640 bp
H/T125-A2
polyA synthétique
T3
néo
SV40 Late Poly-A
Ori f1
Xba I 2538
Sal I 2833
Promoteur SV40
Xho I 2841
Digestion Xho I + Sal I
IE enhancer CMV
EcoR I 1095
IE promoteur CMV
intron
promoteur T7
Amp résistance
H/T125-A2
polyA synthétique
T125-IG24
9058 bp
néo
promoteur T3
SV40 Late Poly-A
Sal I 2833
SV40 ori
IE enhancer CMV
SV40 Enh-prom préc
IE promoteur CMV
Ori f1
intron
promoteur T3
promoteur T7
Xho I 5269
SV40 Late Poly-A
K/T125-A2
EcoR I 3951
EcoR I 4677

FIGURE 5

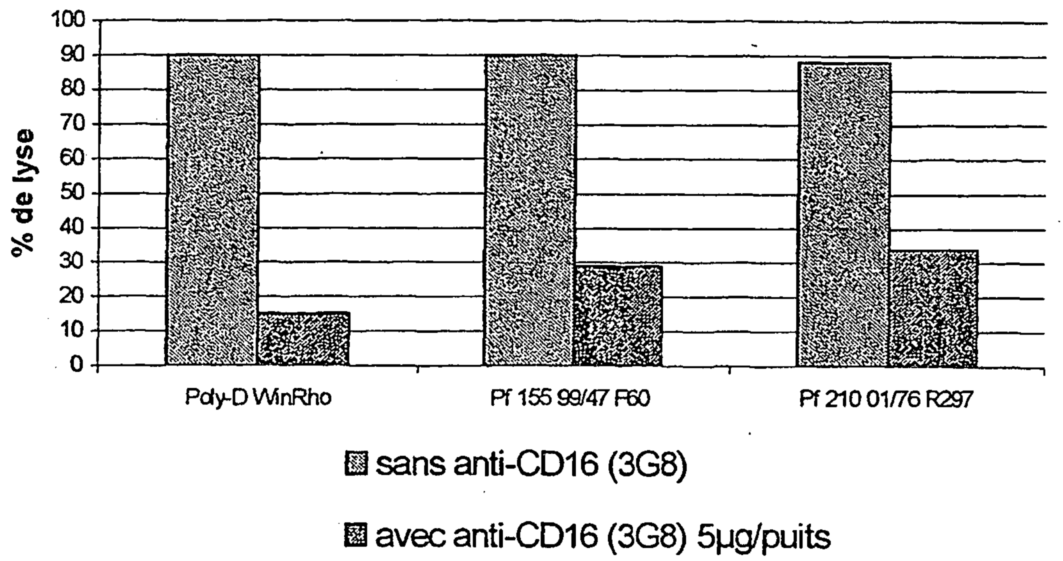
100
90
80
70
60
50
40
30
20
10
0
% de lyse
Poly-D WinRho
Pf 155 99/47 F60
Pf 210 01/76 R297
sans anti-CD16 (3G8)
avec anti-CD16 (3G8) 5µg/puits

FIGURE 6

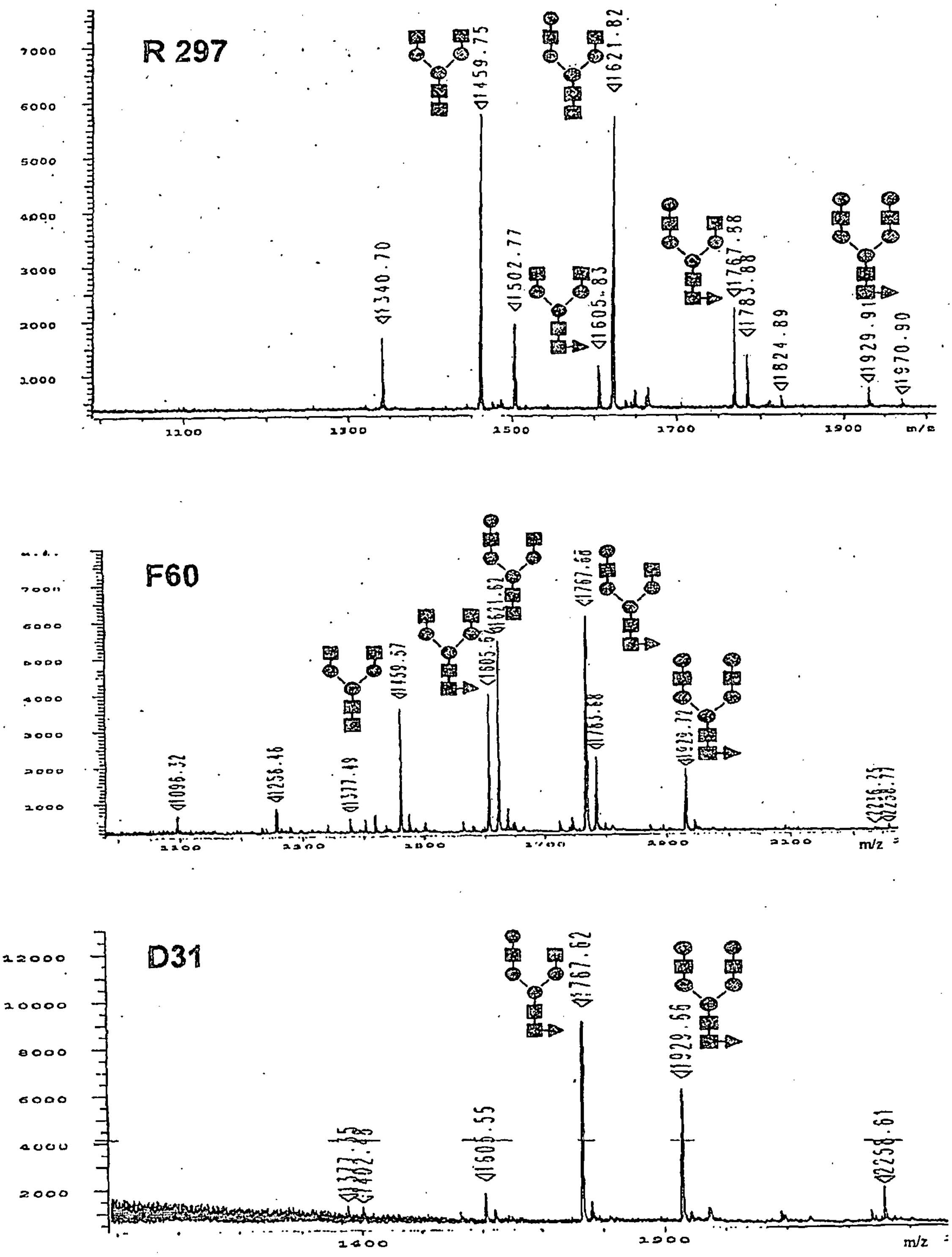


Figure 7

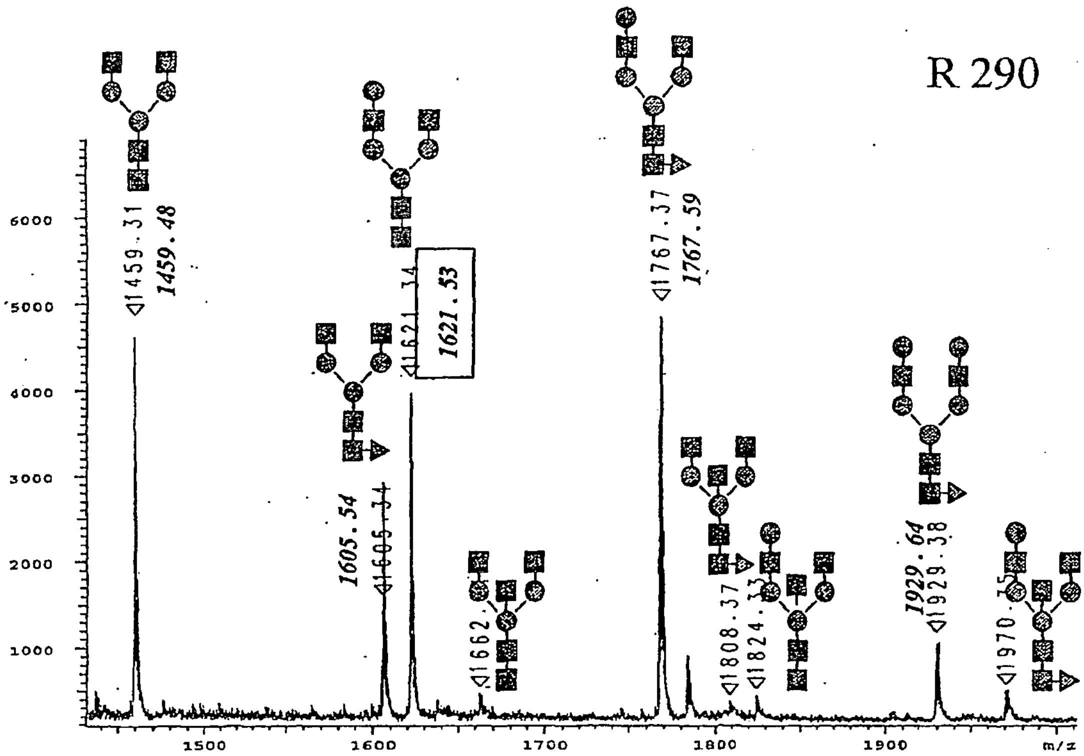
R 290
6000
5000
4000
3000
2000
1000
1500
1600
1700
1800
1900
m/z
1459.31
1459.48
1605.54
1605.34
1621.34
1621.53
1662
1767.37
1767.59
1808.37
1824.33
1929.64
1929.38
1970.75

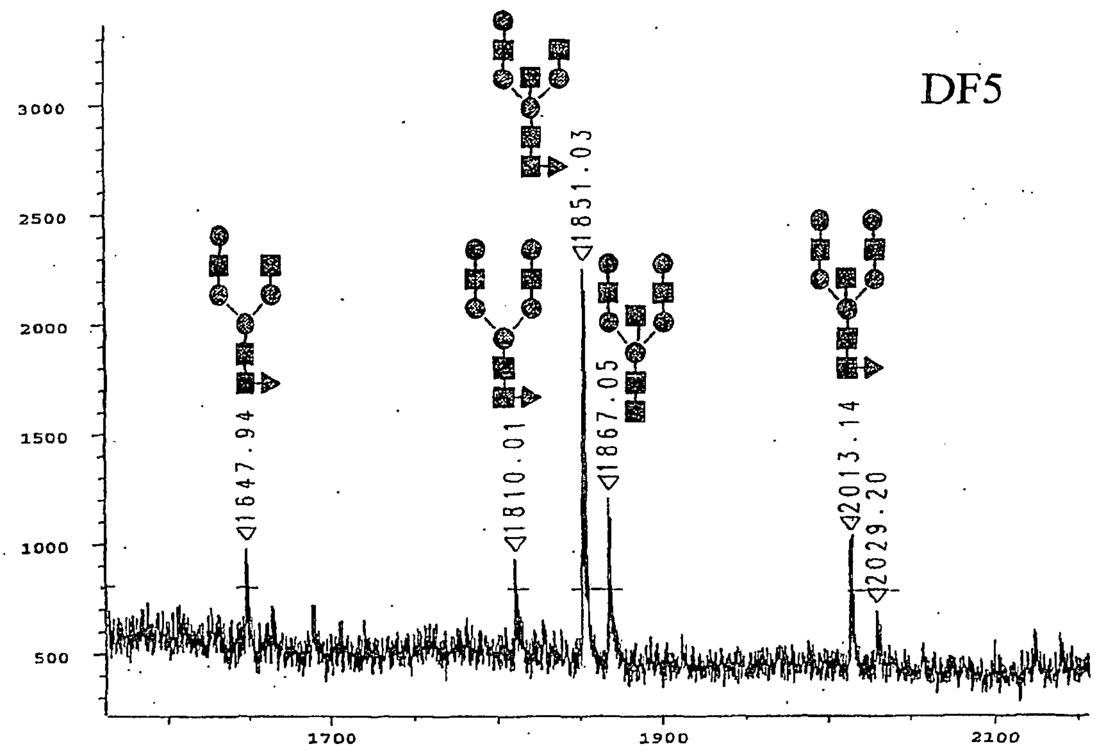
DF5
3000
2500
2000
1500
1000
500
1700
1900
2100
1647.94
1810.01
1851.03
1867.05
2013.14
2029.20

Figure 8

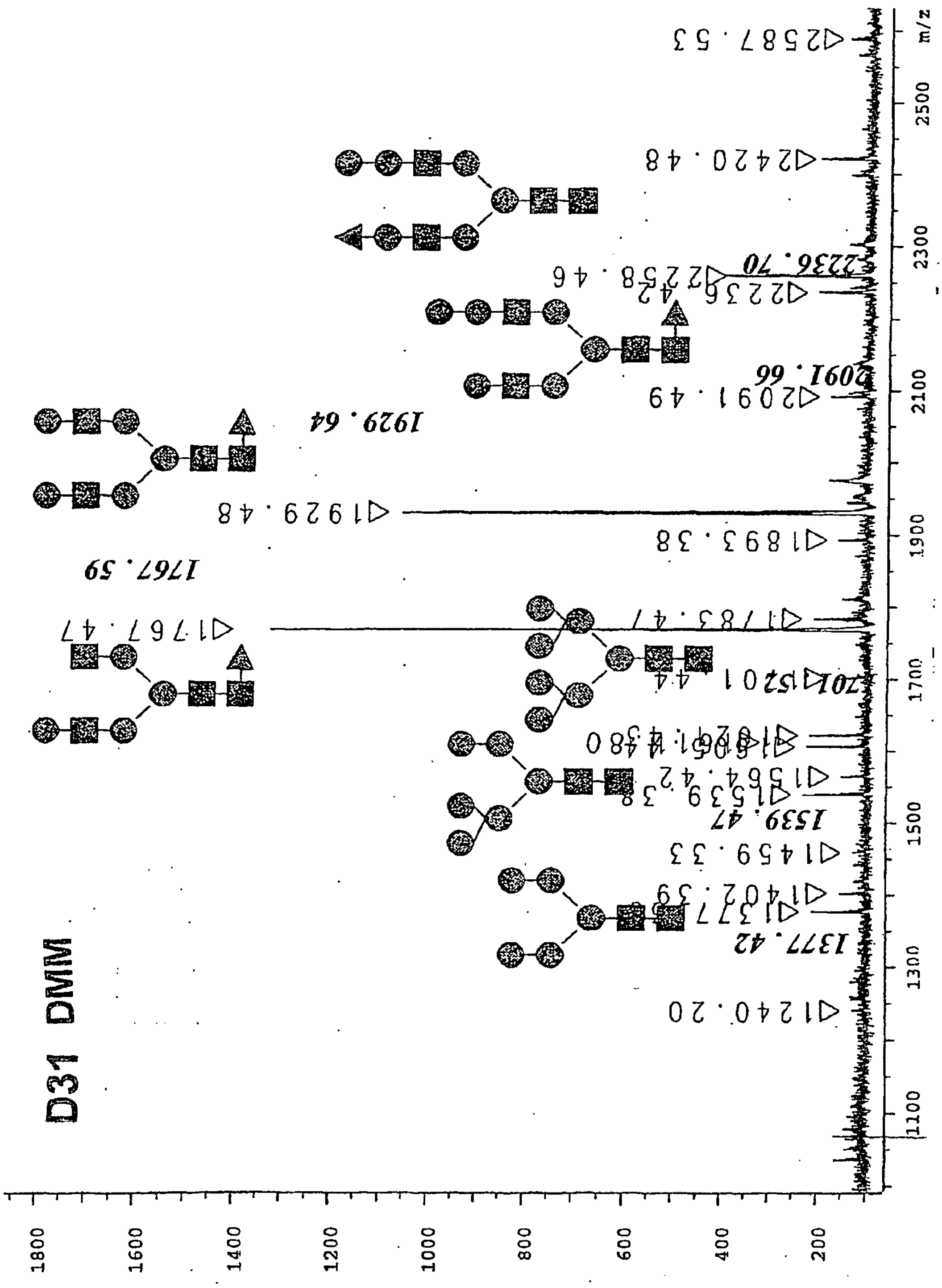
D31 DMM
1240.20
1377.42
1402.59
1459.33
1539.47
1539.38
1564.42
1701
1701.44
1767.47
1767.59
1783.47
1893.38
1929.48
1929.64
2091.49
2091.66
2236.42
2236.70
2258.46
2420.48
2587.53
m/z

Figure 9

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 576093 A
- RU 2094462
- WO 8502413 A
- GB 8610106 A
- EP 0251440 A
- WO 8902442 A
- WO 8902600 A
- WO 89024443 A
- WO 8607740 A
- JP 63050710 A
- JP 58248865 A
- CA 82406033
- GB 8226513 A
- FR 9207893
- GB 2189506 A

### Littérature non-brevet citée dans la description


- **KLEIN P. et al.** *Human antibodies,* 1997, vol. 8 (1), 17-25
- **HADLEY A. et al.** *Immunology,* 1992, vol. 76 (5), 446-51
- Human Monoclonal Antibody against Rh (D) Antigen and its Uses. Board of Trustees of the Leland Stanford Jr. University
- Production of heterohybridomas for manufacture of human monoclonal antibodies to Rhesus D antigen. Central Blood Laboratories Authority
- Human Anti-Rhesus D Producing Heterohybridomas. Central Blood Laboratories Authority
- Human Anti-Rh (D) Monoclonal Antibodies. Central Blood Laboratories Authority
- Protein Performance SA. Institut Pasteur
- Reagents for Determination of Blood Group Substance Rh (D) Factor. International Reagents Corp,
- Japan, Preparation of Monoclonal Antibody to Rh (D) positive Antigen. Mitsubishi Chemical Industries Co., Ltd,
- Human Monoclonal Antibodies. Queens University at Kingston
- Human Monoclonal Antibody against Rhesus D Antigen. University College London
- **FENOUILLET et al.** *Virology,* 1996, vol. 218, 224-231
- **BOYLSTON, J.M. ; GARDNER, B. ; ANDERSON, R.L. ; HUGHES-JONES, N.C.** Production of human IgM anti-D in tissue culture by EB virus-transformed lymphocytes. *Scand. J. Immunol.,* 1980, vol. 12, 355-358
- **BRON, D. ; FEINBERG, M.B. ; TENG, N.N.H. ; KAPLAN, H.S.** Production of Human Monoclonal IgG Antibodies against Rhesus (D) Antigen. *Proc. Nat. Acad. Sci. USA,* 1984, vol. 81, 3214-3217
- **CHOUCHANE, L. ; VAN SPRONSEN, A. ; BREYER, J. ; GUGLIELMI, P. ; STROSBERG, AD.** Molecular characterization of a human anti-Rh(D) antibody with a DH segment encoded by a germ-line sequence. *Eur. J. Biochem.,* 1992, vol. 1 ;207 (3), 1115-1121
- **CRAWFORD, D.H. ; BARLOW, M.J. ; HARRISON, J.F. ; WINGER, L. ; HUEHNS, E.R.** Production of human monoclonal antibody to rhesus D antigen. *Lancet,* 1983, vol. i, 386-388
- **DOYLE, A. ; JONES, T.J. ; BIDWELL, J.L ; BRADLEY, B.A.** In vitro development of human monoclonal antibody secreting plasmacytomas. *Hum. Immunol.,* 1985, vol. 13, 199-209
- **EDELMAN, L. ; MARGARITTE, C. ; CHAABIHI, H. ; MONCHÂTRE, E. ; BLANCHARD, D. ; CARDONA, A. ; MORIN, F. ; DUMAS, G. ; PETRES, S. ; KACZOREK, M.** Obtaining a functional recombinant anti-rhesus (D) antibody using the baculovirus-insect cell expression System. *Immunology,* 1997, vol. 91 (1), 13-19
- **FOUNG, S.K.H. ; BLUNT, J.A. ; WU, P.S. ; AHEARN, P. ; WINN, L.C. ; ENGLEMAN, E.G. ; GRUMET, F.C.** Human Monoclonal Antibodies to Rho (D). *Vox Sang.,* 1987, vol. 53, 44-47
- **GOOSSENS, D. ; CHAMPOMIER, F. ; ROUGER, P. ; SALMON, C.** Human Monoclonal Antibodies against Blood Group Antigens : Preparation of a series of stable EBV immortalized B clones producing high levels of antibody of different isotypes and specificities. *J. Immunol. Methods,* 1987, vol. 101, 193-200
- **ISSITT, P.D.** Genetics of the Rh Blood Group System : Some Current Concepts. *Med. Lab. Sci.,* 1988, vol. 45, 395-404

- **JEFFERIS, R ; LUND, J. ; MIZUTANI, H. ; NAKAGAWA, H. ; KAWAZOE, Y. ; ARATA, Y. ; TAKAHASHI, N.** A comparative study of the N-linked oligosaccharides structure of human IgG Subclass proteins. *Biochem. J.,* 1990, vol. 268, 529-537
- **KOSKIMIES, S.** Human Lymphoblastoid Cell Line Producing Specific Antibody against Rh-Antigen D. *Scand. Immunol.,* 1980, vol. 11, 73-77
- **KUMPEL, B.M. ; GOODRICK, M.J. ; PAMPHILON, D.H. ; FRASER, I.D. ; POOLE G.D. ; MORSE, C. ; STANDEN, G.R. ; CHAPMAN, G.E. ; THOMAS, D.P. ; ANSTEE, D.J.** Human Rh D monoclonal antibodies (BRAD-3 and BRAD-5) Cause Accelerated Clearance of Rh D + Red blood Cells and Suppression of Rh D Immunization in Rh D Volunteers. *Blood,* 1995, vol. 86 (5), 1701-1709
- **KUMPEL, B.M. ; POOLE, G.D. ; BRADLEY, B.A.** Human Monoclonal Anti-D Antibodies. I. Their Production, Serology, Quantitation and Potential Use as Blood Grouping Reagents. *Brit. J. Haemat.,* 1989, vol. 71, 125-129
- **KUMPEL, B.M. ; RADEMACHER, T.W. ; ROOK, G.A.W. ; WILLIAMS, P.J. ; WILSON, I.B.M.** Galactosylation of human IgG anti-D produced by EBV-transformed B lymphoblastoid cell lines is dependent on culture method and affects Fc receptor mediated functional activity. *Hum. Antibodies and Hybridomas,* 1994, vol. 5, 143-151
- **LEATHERBARROW, R.J. ; RADEMACHER, T.W. ; DWEK, R.A. ; WOOF, J.M. ; CLARK, A. ; BURTON, D.R. ; RICHARDSON, N. ; FEINSTEIN, A.** Effector functions of monoclonal aglycosylated mouse IgG2a ; binding and activation of complement component C1 and interaction with human Fc receptor. *Molec. Immun.,* 1985, vol. 22, 407-415
- **LOMAS, C. ; TIPPETT, P. ; THOMPSON, K.M. ; MELAMED, M.D. ; HUGHES-JONES, N.C.** Demonstration of seven epitopes on the Rh antigen D using human monoclonal anti-D antibodies and red cells from D categories. *Vox Sang.,* 1989, vol. 57, 261-264
- **LUND, J. ; TAKAHASKI, N. ; NAKAGAWA, H. ; GOODALL, M. ; BENTLEY, T. ; HINDLEY, S.A. ; TYLER, R. ; JEFFERIS, R.** Control of IgG/Fc glycosylation : a comparison of oligosaccharides from chimeric human/mouse and mouse subclass immunoglobulin G5. *Molec. Immun.,* 1993, vol. 30 (8), 741-748
- **LUND, J. ; TANAKA, T. ; TAKAHASHI, N. ; SARMAY, G. ; ARATA, Y. ; JEFFERIS, R.** A protein structural change in aglycosylated IgG3 correlates with loss of hu Fc□RI and hu FcγRIII binding and/or activation. *Molec. Immun.,* 1990, vol. 27, 1145-1153
- **MA, J.K. ; HEIN, M.B.** Immunotherapeutic potential of antibodies produced in plants. *Trends Biotechnol.,* 1995, vol. 13, 522-527
- **MC CANN-CARTER, M.C. ; BRUCE, M. ; SHAW, E.M. ; THORPE, S.J. ; SWEENEY, G.M. ; ARMSTRONG, S.S. ; JAMES, K.** The production and evaluation of two human monoclonal anti-D antibodies. *Transf. Med.,* 1993, vol. 3, 187-194
- **MELAMED, M.D. ; GORDON, J. ; LEY, S.J. ; EDGAR, D. ; HUGHES-JONES, N.C.** Senescence of a human lymphoblastoid clone producing anti-Rhesus (D). *Eur. J. Immunol.,* 1985, vol. 115, 742-746
- **PAREKH, R.B. ; DWEK, R.A. ; SUTTON, B.J. ; FERNANDES, D.L. ; LEUNG, A. ; STANWORTH, D. ; RADEMACHER, T.W. ; MIZUOCHI, T. ; TANIGUCHI, T. ; MATSUTA, K.** Asssociation of rheumatoid arthritis and primary osteoarthritis with changes in the glycosylation pattern of total serum IgG. *Nature,* 1985, vol. 316, 452-457
- **ROTHMAN, RJ. ; PERUSSIA, B. ; HERLYN, D. ; WARREN, L.** Antibody-dependent cytotoxicity mediated by natural killer cells is enhanced by castanospermine-induced alterations of IgG glycosylation. *Mol. Immunol.,* 1989, vol. 26 (12), 1113-1123
- **SHITARA K. ; NAKAMURA K. ; TOKUTAKE-TANAKA Y. ; FUKUSHIMA M. ; HANAI N.** A new vector for the high level expression of chimeric antibodies to myeloma cells. *J. Immunol. Methods,* 1994, vol. 167, 271-278
- **THOMPSON, K.M. ; HOUGH, D.W. ; MADDISON, P.J. ; MELAMED, M.D. ; HUGHES-JONES, N.C.** Production of human monoclonal IgG and IgM antibodies with anti-D (rhesus) specificity using heterohybridomas. *Immunology,* 1986, vol. 58, 157-160
- **THOMSON, A. ; CONTRERAS, M. ; GORICK, B. ; KUMPEL, B. ; CHAPMAN, G.E. ; LANE, R.S. ; TEESDALE, P. ; HUGHES-JONES, N.C. ; MOLLISON, P.L.** Clearance of Rh D-positive red cells with monoclonal anti-D. *Lancet,* 1990, vol. 336, 1147-1150
- **TIPPETT, P.** Sub-divisions of the Rh (D) antigen. *Med. Lab. Sci.,* 1988, vol. 45, 88-93
- **WARE, R.E. ; ZIMMERMAN, S.A.** Anti-D : Mechanisms of action. *Seminars in Hematology,* 1998, vol. 35 (1), 14-22
- **YU, I.P.C. ; MILLER, W.J. ; SILBERKLANG, M. ; MARK, G.E. ; ELLIS, R.W. ; HUANG, L. ; GLUSHKA, J. ; VAN HALBEEK, H. ; ZHU, J. ; ALHADEFF, J.A.** Structural characterization of the N-Glycans of a humanized anti-CD18 murine immunoglobulin G. *Arch. Biochem. Biophys.,* 1994, vol. 308, 387-399
- **ZUPANSKA, B. ; THOMPSON, E. ; BROJER, E. ; MERRY, A.H.** Phagocytosis of Erythrocytes Sensitized with Known Amounts of IgG1 and IgG3 anti-Rh antibodies. *Vox Sang.,* 1987, vol. 53, 96-101